# EUROPEAN PATENT SPECIFICATION

(11) **EP 2 831 110 B1**
(45) Date of publication and mention of the grant of the patent: **09.05.2018**
(21) Application number: 13770310.4
(22) Date of filing: 14.03.2013
(51) Int. Cl.: C07K 16/00, A61K 39/395, C07K 16/30, A61K 39/00, A61K 45/06, C07K 16/18, C07K 16/22, C07K 16/28, G01N 33/574

(54) **ANTI-EMP2 THERAPY REDUCES CANCER STEM CELLS**
ANTI-EMP2-THERAPIE ZUR REDUKTION VON KREBSSTAMMZELLEN
TRAITEMENT ANTI-EMP2 RÉDUISANT LES CELLULES SOUCHES CANCÉREUSES

(30) Priority: 30.03.2012 US 201261617996 P
(43) Date of publication of application: 04.02.2015
(73) Proprietor: The Regents of the University of California, Oakland, CA 94607-5200 (US); Paganini Biopharma, Inc., Encino, California 91316-3904 (US)
(72) Inventor: WADEHRA, Madhuri, Manhattan Beach, CA 90266 (US); BRAUN, Jonathan, Tarzana, CA 91356 (US); GORDON, Lynn, K., Tarzana, CA 91356 (US); LAZAR, Gary, S., Encino, CA 91316 (US)
(74) Representative: Murgitroyd & Company
(86) International application number: PCT/US2013/031542
(87) International publication number: WO 2013/148263

(56) References cited:
- WO-A2-2009/048980
- WO-A2-2013/056248
- US-A1- 2009 123 439
- US-A1- 2010 272 732
- US-A1- 2010 272 732
- DATABASE UNIPROTKB [Online] 01 October 1996 XP003032696 Database accession no. P54851

## Description

### CROSS REFERENCE TO RELATED APPLICATIONS

This application claims the benefit under 35 U.S.C. § 119(e) to U.S. Application No. 61/617,996 filed March 30, 2012, the disclosure of which is incorporated by reference in its entirety.

### GOVERNMENT RIGHTS

This work was supported by the U.S. Department of Veterans Affairs, and the Federal Government has certain rights in this invention.

### FIELD OF THE INVENTION

This invention relates to anti-EMP2 antibodies, their pharmaceutical compositions and methods for using them to reduce and detect cancer stem cells in multiple types of cancer. More specifically, the invention also relates to methods of identifying cancer stem cells, target/drug discovery, anti-tumor vaccines, and cancer diagnosis and treatment.

### BACKGROUND

Cancer fatalities in the United States alone number in the hundreds of thousands each year and cancer remains a major cause of mortality worldwide. Despite advances in the treatment of certain forms of cancer through surgery, radiotherapy, and chemotherapy, many types of cancer remain essentially incurable. Even when an initial bout of cancer appears to be effectively treated by surgical removal, radiation, and/or chemotherapy, the cancer commonly reoccurs. Such recurrent cancers become highly resistant or refractory to chemotherapeutics. Such rapid recurrence and refractoriness, after chemotherapy, are considered to be caused by cancer stem cells (CSCs).

CSCs are cancer cells that have the common characteristics of normal stem cells. Specifically, like all stem cells, CSCs have the capacity to self renew and to differentiate into multiple lineages. Accordingly, CSCs can differentiate into cancer cells (*i.e*., the CSCs are tumorigenic).

CSCs comprise a fraction of tumor cells with stem cell-like properties, such as the ability to initiate and maintain neoplastic clones. These cells have the ability to self-renew, but also give rise to progenitors that yield phenotypically diverse cancer cells but with lower tumorigenic potential. This subpopulation of stem cell-like cells are the ones that are efficient at tumor formation and metastatic tumor spread as compared to tumor cells that are not cancer stem cells.

Over the last few years, tremendous progress has been made in the recognition and understanding of cancer stem cells (CSC). It is now accepted that the activation of specific pathways can confer "stem cell-like" properties on a subset of tumor cells. CSC have the ability to self-renew or differentiate into additional "daughter" cells, and they are thought to be the major drivers for tumor recurrence and metastasis(1). CSCs are of particular concern to new drug development as these cells are not eliminated by conventional therapy but in fact enriched. Thus, identifying new targets and drugs to eliminate these cells are crucial for patient care.

CSCs are a prerequisite for many types of cancer ontogenesis. Cancer stem cells exhibit low proliferative rates, high self-renewing capacity, a propensity to differentiate into actively proliferating tumor cells, and show resistance to chemotherapy or radiation (*see e.g.* Van der Griend et al. 2008). Furthermore, CSCs have been identified in a wide variety of cancers including, for example, blood, breast, brain, colon, melanoma, pancreatic, prostate, ovarian, and lung cancers. Specifically, CSCs can be found in leukemias, glioblastomas, medulloblastomas, and almost all types of epithelial tumors (carcinomas). Accordingly, CSCs likely play a role tumor growth, cancer progression, metastases, and reoccurrence in a wide variety of cancers.

A number of molecules have been identified on cancer stems including CD44+, CD24-, ESA+ and ALDH1 expression, but these proteins remain unattractive targets as they are broadly expressed (Lobo et al., "The Biology of Cancer Stem Cells," Annual Review of Cell and Developmental Biology. 2007;23:675-99; Charafe-Jauffret et al., "Breast Cancer Cell Lines Contain Functional Cancer Stem Cells with Metastatic Capacity and a Distinct Molecular Signature," Cancer Research, 2009;69:1302-13; Biddle et al., "Cancer Stem Cells in Squamous Cell Carcinoma Switch between Two Distinct Phenotypes That Are Preferentially Migratory or Proliferative," Cancer Research, 2011;71:5317-26). Moreover, it has been shown that cancer stem cells are relatively resistant to both radiation and chemotherapy, thus significantly contributing to resistance and relapse following therapy (Charafe-Jauffret et al., "Breast Cancer Cell Lines Contain Functional Cancer Stem Cells with Metastatic Capacity and a Distinct Molecular Signature," Cancer Research, 2009;69:1302-13; Biddle et al., "Cancer Stem Cells in Squamous Cell Carcinoma Switch between Two Distinct Phenotypes That Are Preferentially Migratory or Proliferative," Cancer Research, 2011;71:5317-26; Li et al., "Intrinsic Resistance of Tumorigenic Breast Cancer Cells to Chemotherapy," Journal of the National Cancer Institute, 2008;100:672-9; Croker et al., "Inhibition of aldehyde dehydrogenase (ALDH) activity reduces chemotherapy and radiation resistance of stem-like ALDHhiCD44+ human breast cancer cells," Breast Cancer Research and Treatment, 2012;133:75-87; Rich et al., "Chemotherapy and Cancer Stem Cells," Cell Stem Cell 2007;1:353-5). In fact, chemotherapy agents such as Paclitaxel and Epirubicin have been shown to increase the number of ALDH positive cells (Tanei et al., "Association of Breast Cancer Stem Cells Identified by Aldehyde Dehydrogenase 1 Expression with Resistance to Sequential Paclitaxel and Epirubicin-Based Chemotherapy for Breast Cancers," Clinical Cancer Research, 2009;15:4234-41).

CSCs can be characterized based on the investigation of distinct surface marker patterns within primary tumors. Among an ever increasing number of proposed biomarkers, CD44, CD133, ABCG2, and ALDH have been used to identify CSCs. Furthermore, aberrant signal pathways are another proposed feature of CSCs. (Hu et al., Am J Cancer Res, 2012, 2(3):340-356). For example, Wnt, Notch, and Hedgehog signaling pathways are proposed features of CSCs.

CD44 was reported as a robust marker of CSCs (Chu et al. 2009; Takaishi et al. 2009). A single CD44+ cell from a colorectal tumor could form a sphere in vitro and was able to generate a xenograft tumor resembling the properties of the primary tumor (Du et al. 2008). CD 133 is also a marker of CSCs. CD 133 was initially described as a surface antigen specific for human hematopoietic stem cells and as a marker for murine neuroepithelia and several other embryonic epithelia (Singh et al. 2004). Some studies have used CD133, alone or in combination with other markers, to isolate CSCs from malignant tumors of colon, lung and liver (Haraguchi et al. 2008). Furthermore, CD133+ tumor cells repair radiation-induced DNA damage more effectively than CD 133- tumor cells (Bao et al. 2006).

CSCs were first reported in human acute myeloid leukemia (AML). (Hu et al., Am J Cancer Res, 2012, 2(3):340-356 and Lapidot et al., Nature, 1994, 367:645-648). There is less than one in 10,000 CSCs in an AML sample. However, even at the rate of 1:10,000, CSCs had the ability to repopulate the AML cells, thereby providing evidence of the CSCs' ability to self-renew and differentiate. Since this first report of CSCs in AML, CSCs have also been identified in solid tumors. For example, the first report of CSCs in solid tumors was in breast cancer in 2003 and later studies have also shown CSCs in brain, colon, melanoma, pancreatic, prostate, ovarian, lunch, and gastric caners. Hu et al., Am J Cancer Res, 2012, 2(3):340-356 and Al-Hajj et al., PNAS, 2003, 100:3983-3988). Accordingly, CSCs are pervasive in a variety of cancers and treatments that target and eradicate CSCs are needed.

The presence of cancer stem cells has profound implications for cancer therapy. Existing therapies have been developed largely against the bulk population of tumor cells, because the therapies are identified by their ability to shrink the tumor mass. However, CSCs are often resistant to chemotherapy and can account for chemotherapy failure (Sell et al. 2008). Therefore, conventional chemotherapies that kill the bulk of cancer cells often leave behind CSCs that are resistant to the conventional chemotherapy. Thus, because CSCs can grow faster after reduction of non-CSC cancer cells by chemotherapy, CSCs are considered to be one of the mechanisms for the quick relapse and reoccurance after chemotherapies.

Furthermore, CSCs arise from a number of different sources. For example, CSCs may arise from random mutations to normal adipose-derived stromal cells, progenitor cells, differentiated cells, and normal stem cells. Normal stem cells are prime targets of CSC progenitors. This is because normal stem cells, like CSCs have the capacity for self-renewal and would theoretically require fewer mutations to transform into CSCs. Furthermore, it has been hypothesized that normal stem cell derived CSCs are the most aggressive of CSCs. (Park et al., Mol Ther. 2009, 17:219-230).

Acordingly, since CSCs by virtue of their relative resistance to chemotherapy and radiation therapy may contribute to treatment resistance and relapse, the successful targeting of this cell population is critical. Strategies designed to specifically target CSC represent an important approach to improving patient outcome. Thus, it is highly desirable to be able to identify further suitable cancer stem cells markers, and to use these markers for diagnostic and prognostic methods and/or for developing therapies that target CSCs.

Therefore, identifying new molecular targets expressed on CSC remains a need in the art. The instant disclosure addresses this need and others.

As reported herein, the epithelial membrane protein-2 (EMP2) is overexpressed in CSCs. EMP2 is a tetraspan protein belonging to the growth arrest specific-3 (GAS3) family. Functionally, EMP2 associates with and modulates the localization and activity of both integrin αvβ3 and focal adhesion kinase (FAK). EMP2 (SEQ ID NO:1) is expressed at high levels in epithelial cells of the lung, eye, and genitourinary tracts. Like several tetraspan proteins (CD9, CD81, PMP22), EMP2 in murine fibroblasts is localized to lipid raft domains. EMP2 controls cell surface trafficking and function of certain integrins, GPI-linked proteins, and class I MHC molecules, and reciprocally regulates caveolin expression. (see, Claas et al., J Biol Chem 276:7974-84 (2001); Hasse et al., JNeurosci Res 69:227-32 (2002); Wadehra et al., Exp Mol Pathol 74:106-12 (2003); Wadehra et al., Mol Biol Cell 15:2073-2083 (2004); Wadehra et al., J Biol Chem 277:41094-41100 (2002); and Wadehra et al., Clin Immunol 107:129-136 (2003)).

SEQ ID NO:1 (ACCESSION P54851) MLVLLAFIIA FHITSAALLF IATVDNAWWV GDEFFADVWR ICTNNTNCTV INDSFQEYST LQAVQATMIL STILCCIAFF IFVLQLFRLK QGERFVLTSI IQLMSCLCVM IAASIYTDRR EDIHDKNAKF YPVTREGSYG YSYILAWVAF ACTFISGMMY LILRKRK

EMP2 appears to regulate trafficking of various proteins and glycolipids by facilitating transfer of molecules from post-Golgi endosomal compartments to appropriate plasma membrane locations. Specifically, EMP2 is thought to facilitate the appropriate trafficking of select molecules into glycolipids-enriched lipid raft microdomains (GEMs) (Wadehra et al., Mol Biol Cell 15:2073-83 (2004)). GEMs are cholesterol rich microdomains which are often associated with chaperones, receptosomes, and protein complexes that are important for efficient signal transduction (Leitinger et al., J Cell Sci 115:963-72 (2002); Moffett et al., J Biol Chem 275:2191-8 (2000)). Moreover, GEMs are involved in correct sorting of proteins from the Golgi apparatus to plasma membrane (Abrami et al., J Biol Chem 276:30729-36 (2001); Galbiati et al., Cell 106:403-11 (2001); Gruenberg et al., Curr Opin Cell Biol 7: 552-63 (1995)). In this respect, modulation of EMP2 expression levels or its location on the plasma membrane alters the surface repertoire of several classes of molecules including integrins, focal adhesion kinase, class I major histocompatibility molecules and other immunoglobulin super-family members such as CD54 and GPI-linked proteins (Wadehra et al., Dev Biol 287:336-45 (2005); Wadehra et al., Clinical Immunology 107:129-36 (2003); Morales et al., Invest Opthalmol Vis Sci (2008)).

EMP2 expression is associated with EMP2 neoplasia (Wadehra et al., Cancer 107:90-8 (2006)). In endometrial cancer, for example, EMP2 is an independent prognostic indicator for tumors with poor clinical outcome. EMP2 positive tumors, compared to EMP2 negative tumors, had a significantly greater myometrial invasiveness, higher clinical state, recurrent or persistent disease following surgical excision, and earlier mortality. WO2009/048980A2 and US2010/0272732A1 both describe methods and compositions for the treatment or prevention of *Chlamydia* infection and cancer. The methods and compositions target cancers which express or overexpress EMP2 nucleic acids and polypeptides by targeting EMP2.

Based on studies described herein it is now shown that EMP2 can be used as a target in the treatment of CSCs in a variety of cancers (e.g., breast cancers). Accordingly EMP2 polypeptides, anti-EMP2 antibodies, and EMP2 siRNA can be used to diagnose and treat CSCs and promote cures for a variety of cancers. As discussed above, there remains a large need for methods and compositions which are useful in the prevention, treatment, and modulation CSCs in cancers. Accordingly, this invention provides novel compositions and methods for meeting these and other needs.

### BRIEF SUMMARY OF THE INVENTION

In one embodiments, this invention comprises a method of reducing the rate of reoccurrence of a cancer in a patient. In certain embodiments, the method comprises detecting cancer stem cells in a patient. In certain embodiments, the cancer stem cells express EMP2 and one or more markers selected from the group consisting of CD44, CD133 ABCG2, and ALDH. In certain embodiments, after cancer stem cells have been detected, a patient is administered an effective amount of an anti-EMP2 antibody. In certain embodiments, the antibody specifically binds to an epitope in the second extracellular loop of EMP2. In certain embodiments, the epitope comprises the amino acid sequence DIHDKNAKFYPVTREGSYG.

In certain embodiments, the antibody further comprises a physiological acceptable carrier or a pharmaceutically acceptable carrier. In certain embodiments, the antibody competes with an antibody comprising the heavy and light chain variable regions of a KS49, a KS41, a KS83, or a KS89 diabody. In certain embodiments, the antibody shares 90% amino acid identity with heavy and light chain variable regions of a KS49, a KS41, a KS83, or a KS89 diabody. In certain embodiments, the antibody comprises CDR sequences identical to those of a KS49, a KS41, a KS83, or a KS89 diabody.

In certain embodiments, the method further comprises administering to the patient an effective amount of at least one additional anti-cancer agent. In certain embodiments, the at least one additional anti-cancer agent is selected from the group consisting of platinum-based chemotherapy drugs, taxanes, tyrosine kinase inhibitors, anti-EGFR antibodies, anti-ErbB2 antibodies, and combinations thereof.

In certain embodiments, the at least one additional anti-cancer agent comprises an EGFR inhibitor. In certain embodiments, the EGFR inhibitor comprises an anti-EGFR antibody. In certain embodiments, the anti-EGFR antibody comprises cetuximab. In certain embodiments, the anti-EGFR antibody is selected from the group consisting of matuzumab, panitumumab, and nimotuzumab. In certain embodiments, the EGFR inhibitor is a small molecule inhibitor of EGFR signaling.

In certain embodiments, the small molecule inhibitor of EGFR signaling is selected from the group consisting of gefitinib, lapatinib, canertinib, pelitinib, erlotinib HCL, PKI-166, PD158780, and AG 1478.

In certain embodiments, the at least one additional anti-cancer agent comprises a VEGF inhibitor. In certain embodiments, the VEGF inhibitor comprises an anti-VEGF antibody. In certain embodiments, the anti-VEGF antibody is bevacizumab.

In certain embodiments, the anti-EMP2 antibody is conjugated with an effector moiety. In certain embodiments, the effector moiety is a toxic agent. In certain embodiments, the toxic agent is such as ricin.

In certain embodiments, the treatment comprises blocking invasiveness of the cancer.

In certain embodiments, the anti-EMP2 antibodies are used in vaccine therapies for the cancer.

In certain embodiments, the patient is human or mammal.

In certain embodiments, the cancer is breast cancer. In certain embodiments, the cancer is a cancer selected from a group comprising brain cancer, colon cancer, melanoma, leukemia (e.g., AML), pancreatic cancer, prostate cancer, ovarian cancer, lung cancer, and gastric cancer.

In certain embodiments, the method further comprises a companion diagnostic. In certain embodiments, the companion diagnostic comprises an anti-EMP2 antibody.

In a second embodiment, this invention comprises a method of reducing the rate of reoccurrence of a breast cancer in a patient. In certain embodiments, the method comprises detecting cancer stem cells in a patient. In certain embodiments, the cancer stem cells express EMP2 and one or more markers selected from the group consisting of CD44, CD133 ABCG2, and ALDH. In certain embodiments, after cancer stem cells have been detected, a patient is administered an effective amount of an anti-EMP2 antibody. In certain embodiments, the antibody specifically binds to an epitope in the second extracellular loop of EMP2. In certain embodiments, the epitope comprises the amino acid sequence DIHDKNAKFYPVTREGSYG.

In certain embodiments, the anti-EMP2 antibody further comprises a physiological acceptable carrier or a pharmaceutically acceptable carrier.

In certain embodiments, the anti-EMP2 antibody competes with an antibody comprising the heavy and light chain variable regions of a KS49, a KS41, a KS83, or a KS89 diabody. In certain embodiments, the antibody shares 90% amino acid identity with heavy and light chain variable regions of a KS49, a KS41, a KS83, or a KS89 diabody. In certain embodiments, the antibody comprises CDR sequences identical to those of a KS49, a KS41, a KS83, or a KS89 diabody.

In certain embodiments, the method further comprises administering to the patient an effective amount of at least one additional anti-cancer agent.

In certain embodiments, the at least one additional anti-cancer agent is selected from the group consisting of platinum-based chemotherapy drugs, taxanes, tyrosine kinase inhibitors, anti-EGFR antibodies, anti-ErbB2 antibodies, and combinations thereof.

In certain embodiments, the anti-EGFR antibody comprises cetuximab. In certain embodiments, the anti-EGFR antibody is selected from the group consisting of matuzumab, panitumumab, and nimotuzumab.

In certain embodiments, at least one additional anti-cancer agent is selected from the group consisting of gefitinib, lapatinib, canertinib, pelitinib, erlotinib HCL, PKI-166, PD158780, and AG 1478.

In certain embodiments, the at least one additional anti-cancer agent comprises a VEGF inhibitor.

In a third embodiment, this invention comprises a method of reducing the rate of reoccurrence of a endometrial cancer in a patient. In certain embodiments, the method comprises detecting cancer stem cells in a patient. In certain embodiments, the cancer stem cells express EMP2 and one or more markers selected from the group consisting of CD44, CD133 ABCG2, and ALDH. In certain embodiments, after cancer stem cells have been detected, a patient is administered an effective amount of an anti-EMP2 antibody. In certain embodiments, the antibody specifically binds to an epitope in the second extracellular loop of EMP2. In certain embodiments, the epitope comprises the amino acid sequence DIHDKNAKFYPVTREGSYG.

In certain embodiments, the anti-EMP2 antibody further comprises a physiological acceptable carrier or a pharmaceutically acceptable carrier.

In certain embodiments, the anti-EMP2 antibody competes with an antibody comprising the heavy and light chain variable regions of a KS49, a KS41, a KS83, or a KS89 diabody. In certain embodiments, the anti-EMP2 antibody shares 90% amino acid identity with heavy and light chain variable regions of a KS49, a KS41, a KS83, or a KS89 diabody. In certain embodiments, the anti-EMP2 antibody comprises CDR sequences identical to those of a KS49, a KS41, a KS83, or a KS89 diabody.

In certain embodiments, the method further comprises administering to the patient an effective amount of at least one additional anti-cancer agent.

In certain embodiments, the at least one additional anti-cancer agent is selected from the group consisting of platinum-based chemotherapy drugs, taxanes, tyrosine kinase inhibitors, anti-EGFR antibodies, anti-ErbB2 antibodies, and combinations thereof.

In certain embodiments, the anti-EGFR antibody comprises cetuximab. In certain embodiments, the anti-EGFR antibody is selected from the group consisting of matuzumab, panitumumab, and nimotuzumab.

In certain embodiments, the at least one additional anti-cancer agent is selected from the group consisting of gefitinib, lapatinib, canertinib, pelitinib, erlotinib HCL, PKI-166, PD158780, and AG 1478. In certain embodiments,

In certain embodiments, the at least one additional anti-cancer agent comprises a VEGF inhibitor.

In a fourth embodiment, the invention comprises A method of detecting cancer stem cells. In certain embodiments, the method comprises obtaining a biological sample derived from a human having or suspected of having cancer. In certain embodiments, the method comprises detecting the expression EMP2 and one or more markers selected from the group consisting of CD44, CD133, ABCG2, and ALDH.

In certain embodiments, the EMP2 expression is detected with an antibody comprising the heavy and light chain variable regions of a KS49, a KS41, a KS83, or a KS89 diabody. In certain embodiments, the antibody shares 90% amino acid identity with heavy and light chain variable regions of a KS49, a KS41, a KS83, or a KS89 diabody.

In certain embodiments, the human has or is suspected of having breast cancer. In certain embodiments, the human has or is suspected of having triple negative breast cancer. In certain embodiments, the human has or is suspected of having endometrial cancer.

### BRIEF DESCRIPTION OF THE DRAWINGS

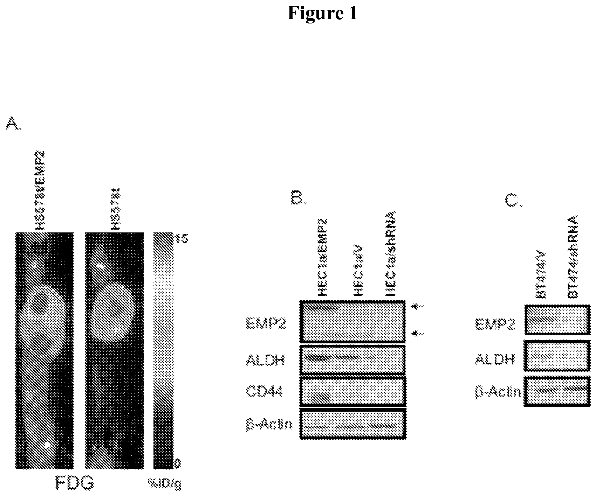

**Figure 1** depicts (A) metabolic analysis by functional positron emission tomography (PET) analysis of HS578t cells in an animal utilizing ¹⁸F-fludeoxyglucose. (B) Analysis of the indicated markers in HEC1a cells trated as described. (C) Analysis of the indicated markers in BT474 cells with and without treatment.
**Figure 2** depicts the results of application of anti-EMP2 antibody on the indicated markers on HCC1937 cells and (B) systemic application of anti-EMP2 antibodies on the indicated xenograft cells.
**Figure 3** depicts experiments that show that anti-EMP2 depletes cancer stem cells in MDA-MB-231 human breast cancer cells.
**Figure 4** depicts experiments that show that anti-EMP2 depletes cancer stem cells in HEC1A human endometrial cancer cells.
**Figure 5** depicts experiments that show that anti-EMP2 + Docetaxel reduces tumor load in MDA-MB-231 human breast cancer cells.

### DETAILED DESCRIPTION OF THE INVENTION

### Introduction

Cancer stem cells (CSCs) are cells within a tumor that have the capacity to self-renew. CSCs also cause the generation of heterogeneous lineages of cancer cells that comprise the tumor. CSCs have been identified in a wide variety of cancers. For example, CSCs have been found in breast brain, colon, melanoma, pancreatic, blood, prostate, ovarian, and lung cancers.

Although CSCs differentiate into cancer cells, the CSCs and the differentiated cancer cells respond differently to common cancer therapies. Specifically, because CSCs are often resistant to chemotherapy and radiation, common cancer therapies target the cancer cells with chemotherapeutics and radiation are not effective at eradicating the CSCs.

Applicants have discovered that EMP2 is expressed in CSCs. Accordingly, in its first aspect, the invention provides compositions of anti-EMP2 antibodies and methods of detecting CSCs in cancers and non-cancer cells. In another aspect, the invention provides compositions of anti-EMP2 antibodies and methods of killing and ablating CSCs in cancers and non-cancer cells. In another aspect, the invention provides compositions of anti-EMP2 antibodies and methods of diagnosing cancers and the likelihood of cancer reoccurance. In a specific aspect, the invention provides the administration of anti-EMP2 antibodies in a physiologically acceptable carrier or a pharmaceutically acceptable carrier. In another aspect, the invention provides compositions of anti-EMP2 antibodies and methods of detecting CSCs in breast cancer. In another aspect, the invention provides compositions of anti-EMP2 antibodies and methods of co-administration with one or more additional therapies. In another aspect, the invention provides companion diagnostic methods and products for use with the methods and antibodies described herein.

For example, it was previously reported that targeting of EMP2 may offer a therapeutic strategy in treating breast cancer, endometrial cancer, and ocular diseases. US Pat. Pubs. 20100272732, 2012026420, 20120020983, and 20100196509, incorporated by reference in their entireties. Aside from anti-EMP2 antibody treatment, common chemotherapeutic drugs used to treat cancers such as breast cancer are anti-VEGF therapies that inhibit angiogenesis. For example, VEGF- therapies include Avastin® (bevacizumab), Sutent® (sunitinib), Lucentis® (ranibizumab), Tykerb® (lapatinib), Nexavar® (sorafenib), axitinib, and pazopanib. However, while these drugs do shrink tumors and slow the time until the cancer progresses, the effect does not last, and the cancer eventually reoccurs, grows, and spreads. Accordingly, while drugs such as Avastin® and Sutent® may kill the breast cancer cells, there is an underlying mechanism that causes regrowth and metastases.

It has been shown that cancer treatments such as Avastin® and Sutent®, *i.e*., treatments that inhbit the growth and formation of blood vessels increase the number of cancer stem cells. (Conley et al., PNAS, 2012, 109(8):2784-2789). Specifically, it was found that in mice that tumors treated with these drugs developed more cancer stem cells, the small number of cells within a tumor that fuel a cancer's growth and spread and that are often resistant to standard treatment. Furthermore, both the number of cancer stem cells and the percentage of cancer stem cells that make up the tumor increased after being treated with each of these therapies. This is a possible explanation for why drugs such as Avastin® and Sutent® may shrink tumor size and slow the progression of recoccurance, but do not prevent tumor reoccurrence and mortality. Accordingly, in order for such drugs to be effective at preventing reoccurance and decreasing mortality, therapies that target and inhibits the survival of CSCs.

Accordingly, the instant disclosure provides anti-EMP2 antibodies that target CSCs. The disclosure further provides method of combining anti-angiogenesis drugs with a anti-EMP2 antibodies to enhance the efficacy of current cancer treatments.

### Breast Cancer

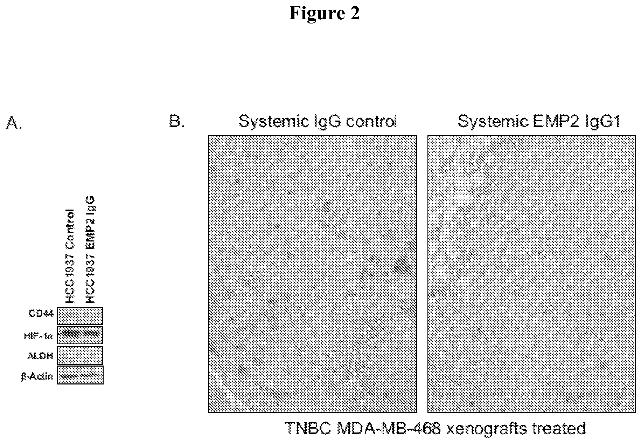

In certain embodiments of this invention, the anti-EMP2 antibodies can be used to target CSCs associated with breast cancer **(****Figures 2 - 5****)**.

A fundamental problem in developing more effective therapeutics to treat breast cancer, including Triple Negative Breast Cancer (TNBC) is the inability of the treatments to affect the viability of breast cancer stem cells which are critical for the development, proliferation and metastasis of breast cancer (Dick JE, "Breast cancer stem cells revealed," PNAS, 2003;100:3547-9). These stem cells make up a very small population of the total cells in tumors. However, like more classical stem cells they have the ability to self-renew, and this property is critical in causing tumor formation, especially during metastasis. A number of studies have indicated that most available anti-cancer agent shrink tumors by killing the more differentiated tumor cells while not impairing the cancer stem cells. The inability of chemotherapeutics to affect the cancer stem cells may be related to the ability of the stem cells to oscillate between active proliferating cells and more quiescent non-dividing cells. Since most chemotherapeutic drugs target dividing and proliferating cells, they may not affect the breast cancer stem cells in their quiescent stage. For example, it has been suggested that the inability of chemotherapeutic drugs to affect breast cancer stem cell survival while at the same time killing differentiated tumor cells may explain why tumor shrinkage may not be a good indicator of patient survival (Liu et al., "Targeting Breast Cancer Stem Cells," Journal of Clinical Oncology, 2010;28:4006-12). While it is accepted that new therapeutics targeting breast cancer stem cells may provide greater efficacy in treating this disease and reduce disease reoccurrence, no drug is currently available that effectively and safely targets and kills these cells.

Breast cancer is the abnormal growth of cells that line the breast tissue ducts and lobules and is classified by whether the cancer started in the ducts or the lobules and whether the cells have invaded (grown or spread) through the duct or lobule, and by the way the cells look under the microscope (tissue histology). It is not unusual for a single breast tumor to have a mixture of invasive and in situ cancer.

Molecular classification of breast cancer has identified specific subtypes, often called "intrinsic" subtypes, with clinical and biological implications, including an intrinsic luminal subtype, an intrinsic HER2-enriched subtype (also referred to as the HER2⁺ or ER⁻/HER2⁺ subtype) and an intrinsic basal-like breast cancer (BLBC) subtype. (Perou et al. 2000). Identification of the intrinsic subtypes has typically been accomplished by a combination of methods, including (1) histopathological detection, (2) ER, PR and HER2 expression status and (3) detection of characteristic cellular markers.

Basal-like breast cancer, which expresses genes characteristic of basal epithelial cells in the normal mammary gland, comprises up to 15%-25% of all breast cancers (Kreike et al. 2007) and is associated with the worst prognosis of all breast cancer types. BLBCs underexpress estrogen receptor (ER⁻), progesterone receptor (PR⁻), and human epidermal growth factor receptor 2 (HER2⁻) and encompass 60% to 90% of so-called "triple-negative" (ER⁻/PR⁻/HER2⁻) breast cancers. Although most basal-like breast cancers are often referred to as triple-negative based on the expression status of ER, PR and HER2, not all basal-like breast cancers are triple negative.

Thus, the intrinsic basal-like breast cancer subtype may be further subdivided into at least three distinct subtypes described herein as "hybrid" basal-like breast cancer subtypes. In addition to a hybrid triple-negative subtype, the hybrid basal-like breast cancer subtypes have profiles that resemble both basal-like breast cancer and at least one other breast cancer molecular subtype. For example, hybrid basal-like subtypes can include a hybrid basal-like/HER2⁺ subtype that has a receptor profile of ER⁻/PR⁻/HER⁺, a hybrid basal-like/luminal subtype that has a receptor profile of ER⁺/PR^{-or+}/HER^{-or+}, and a hybrid basal-like/triple negative subtype that has a receptor profile of ER⁻PR⁻HER⁻.

The intrinsic luminal breast cancer subtype is characterized by expression or overexpression of ER and/or PR (ER⁺ and/or PR⁺). The luminal subtype can be further subdivided based on HER2 status into the luminal A subtype, which is additionally characterized by underexpression of HER2 (ER⁺/PR^{+or-}/HER⁻), and luminal B subtype, which is additionally characterized by overexpression of HER2 (ER⁺/PR^{+or-}/HER⁺). Intrinsic luminal subtypes are often considered to be the most treatable breast cancer subtype and are associated with the best prognosis.

Whereas ER and HER2 guide treatment of luminal and HER2 breast cancers, respectively, chemotherapy remains the only modality of systemic therapy for BLBC. Preferentially affecting younger women, particularly African American women, BLBCs are associated with high histologic grade, aggressive clinical behavior, and a high rate of metastasis to the brain and lung (Carey et al. 2006). Unlike other breast cancer subtypes, there seems to be no correlation between tumor size and lymph node metastasis in BLBCs (Dent et al. 2007).

BLBCs are associated with expression of basal cytokeratins (CK5/6, CK14, and CK17), epidermal growth factor receptor (EGFR), c-kit, and p53 and associated with the absence of ER, PR, and HER2 expression. With a large variety of associated genes, BLBCs have been defined differently in different studies using a set of diagnostic markers. For example, Nielsen et al. defined BLBC on the basis of negative ER and negative HER2 expression in addition to positive basal cytokeratin, EGFR, and/or c-kit expression (Nielsen et al. 2004). On the other hand, other groups have defined BLBC on the basis of on a combination of negative ER, and negative HER2 expression and positive CK5, P-cadherin, and p63 expression (Elsheikh et al. 2008) or positive vimentin, EGFR, and CK5/6 expression (Livasy et al. 2006). These different technical approaches in combination with widely varying patient cohorts may explain the inconsistent experimental results for these markers.

Identification of the basal-like subtype using immunohistochemistry (IHC) for detecting hormone receptors alone is less desirable than detecting a theranostic biomarker, because identification is based on the absence of IHC staining for estrogen receptor (ER), progesterone receptor (PR), and human epidermal growth factor receptor 2 (HER2) rather than the presence of a specific tumor marker or markers. Its diagnosis is more one of exclusion rather than inclusion.

Basal-like breast cancer is often synonymously referred to as "triple negative" (i.e., ER⁻/PR⁻/HER2⁻), however, not all triple negative breast cancers are basal-like, and not all basal-like breast cancers are triple negative. Although other molecular markers have been associated with basal-like breast cancer as described above, such markers are not exclusive to this basal-like breast cancer.

Breast cancer subsets can be treated with antibodies such as those provided herein.

### Antibodies

Antibodies that find use in the present invention can take on a number of formats such as traditional antibodies as well as antibody derivatives, fragments and mimetics. In certain embodiments of this invention, the anti-EMP2 antibodies are KS49, KS41, KS83, or KS89. These antibodies and their use are described herein.

Traditional antibody structural units typically comprise a tetramer. Each tetramer is typically composed of two identical pairs of polypeptide chains, each pair having one "light" (typically having a molecular weight of about 25 kDa) and one "heavy" chain (typically having a molecular weight of about 50-70 kDa). Human light chains are classified as kappa and lambda light chains. Heavy chains are classified as mu, delta, gamma, alpha, or epsilon, and define the antibody's isotype as IgM, IgD, IgG, IgA, and IgE, respectively. IgG has several subclasses, including, but not limited to IgG1, IgG2, IgG3, and IgG4. IgM has subclasses, including, but not limited to, IgM1 and IgM2. Thus, "isotype" as used herein is meant any of the subclasses of immunoglobulins defined by the chemical and antigenic characteristics of their constant regions. The known human immunoglobulin isotypes are IgG1, IgG2, IgG3, IgG4, IgA1, IgA2, IgM1, IgM2, IgD, and IgE. It should be understood that therapeutic antibodies can also comprise hybrids of isotypes and/or subclasses.

The amino-terminal portion of each chain includes a variable region of about 100 to 110 or more amino acids primarily responsible for antigen recognition. In the variable region, three loops are gathered for each of the V domains of the heavy chain and light chain to form an antigen-binding site. Each of the loops is referred to as a complementarity-determining region (hereinafter referred to as a "CDR"), in which the variation in the amino acid sequence is most significant. "Variable" refers to the fact that certain segments of the variable region differ extensively in sequence among antibodies. Variability within the variable region is not evenly distributed. Instead, the V regions consist of relatively invariant stretches called framework regions (FRs) of 15-30 amino acids separated by shorter regions of extreme variability called "hypervariable regions" that are each 9-15 amino acids long or longer.

Each VH and VL is composed of three hypervariable regions ("complementary determining regions," "CDRs") and four FRs, arranged from amino-terminus to carboxy-terminus in the following order: FR1-CDR1-FR2-CDR2-FR3-CDR3-FR4.

The hypervariable region generally encompasses amino acid residues from about amino acid residues 24-34 (LCDR1; "L" denotes light chain), 50-56 (LCDR2) and 89-97 (LCDR3) in the light chain variable region and around about 31-35B (HCDR1; "H" denotes heavy chain), 50-65 (HCDR2), and 95-102 (HCDR3) in the heavy chain variable region; Kabat et al., SEQUENCES OF PROTEINS OF IMMUNOLOGICAL INTEREST, 5th Ed. Public Health Service, National Institutes of Health, Bethesda, Md. (1991) and/or those residues forming a hypervariable loop (e.g. residues 26-32 (LCDR1), 50-52 (LCDR2) and 91-96 (LCDR3) in the light chain variable region and 26-32 (HCDR1), 53-55 (HCDR2) and 96-101 (HCDR3) in the heavy chain variable region; Chothia and Lesk (1987) J. Mol. Biol. 196:901-917. Specific CDRs of the invention are described below.

Throughout the present specification, the Kabat numbering system is generally used when referring to a residue in the variable domain (approximately, residues 1-107 of the light chain variable region and residues 1-113 of the heavy chain variable region) (e.g, Kabat *et al.*, supra (1991)).

The CDRs contribute to the formation of the antigen-binding, or more specifically, epitope binding site of antibodies. "Epitope" refers to a determinant that interacts with a specific antigen binding site in the variable region of an antibody molecule known as a paratope. Epitopes are groupings of molecules such as amino acids or sugar side chains and usually have specific structural characteristics, as well as specific charge characteristics. A single antigen may have more than one epitope. For example, as described herein the antibodies bind to an epitope in the presumptive second extracellular domain of EMP2.

The epitope may comprise amino acid residues directly involved in the binding (also called immunodominant component of the epitope) and other amino acid residues, which are not directly involved in the binding, such as amino acid residues which are effectively blocked by the specifically antigen binding peptide; in other words, the amino acid residue is within the footprint of the specifically antigen binding peptide.

In some embodiments, the epitope is derived from SEQ ID NO:2, wherein SEQ ID NO:2 is EDIHDKNAKFYPVTREGSYG and represents a 20-mer polypeptide sequence from the second extracellular loop of human EMP2

Epitopes may be either conformational or linear. A conformational epitope is produced by spatially juxtaposed amino acids from different segments of the linear polypeptide chain. A linear epitope is one produced by adjacent amino acid residues in a polypeptide chain. Conformational and nonconformational epitopes may be distinguished in that the binding to the former but not the latter is lost in the presence of denaturing solvents.

An epitope typically includes at least 3, and more usually, at least 5 or 8-10 amino acids in a unique spatial conformation. Antibodies that recognize the same epitope can be verified in a simple immunoassay showing the ability of one antibody to block the binding of another antibody to a target antigen, for example "binning."

The carboxy-terminal portion of each chain defines a constant region primarily responsible for effector function. Kabat *et al.* collected numerous primary sequences of the variable regions of heavy chains and light chains. Based on the degree of conservation of the sequences, they classified individual primary sequences into the CDR and the framework and made a list thereof (see SEQUENCES OF IMMUNOLOGICAL INTEREST, 5th edition, NIH publication, No. 91-3242, E.A. Kabat et al., entirely incorporated by reference).

In the IgG subclass of immunoglobulins, there are several immunoglobulin domains in the heavy chain. By "immunoglobulin (Ig) domain" herein is meant a region of an immunoglobulin having a distinct tertiary structure. Of interest in the present invention are the heavy chain domains, including, the constant heavy (CH) domains and the hinge domains. In the context of IgG antibodies, the IgG isotypes each have three CH regions. Accordingly, "CH" domains in the context of IgG are as follows: "CH1" refers to positions 118-220 according to the EU index as in Kabat. "CH2" refers to positions 237-340 according to the EU index as in Kabat, and "CH3" refers to positions 341-447 according to the EU index as in Kabat.

Another type of Ig domain of the heavy chain is the hinge region. By "hinge" or "hinge region" or "antibody hinge region" or "immunoglobulin hinge region" herein is meant the flexible polypeptide comprising the amino acids between the first and second constant domains of an antibody. Structurally, the IgG CH1 domain ends at EU position 220, and the IgG CH2 domain begins at residue EU position 237. Thus for IgG the antibody hinge is herein defined to include positions 221 (D221 in IgG1) to 236 (G236 in IgG1), wherein the numbering is according to the EU index as in Kabat. In some embodiments, for example in the context of an Fc region, the lower hinge is included, with the "lower hinge" generally referring to positions 226 or 230.

Of interest in the present invention are the Fc regions. By "Fc" or "Fc region" or "Fc domain" as used herein is meant the polypeptide comprising the constant region of an antibody excluding the first constant region immunoglobulin domain and in some cases, part of the hinge. Thus Fc refers to the last two constant region immunoglobulin domains of IgA, IgD, and IgG, the last three constant region immunoglobulin domains of IgE and IgM, and the flexible hinge N-terminal to these domains. For IgA and IgM, Fc may include the J chain. For IgG, the Fc domain comprises immunoglobulin domains Cy2 and Cγ3 (Cγ2 and Cγ3) and the lower hinge region between Cγ1 (Cγ1) and Cγ2 (Cγ2). Although the boundaries of the Fc region may vary, the human IgG heavy chain Fc region is usually defined to include residues C226 or P230 to its carboxyl-terminus, wherein the numbering is according to the EU index as in Kabat. In some embodiments, as is more fully described below, amino acid modifications are made to the Fc region, for example to alter binding to one or more FcγR receptors or to the FcRn receptor.

In some embodiments, the antibodies are full length. By "full length antibody" herein is meant the structure that constitutes the natural biological form of an antibody, including variable and constant regions, including one or more modifications as outlined herein.

Alternatively, the antibodies can be a variety of structures, including, but not limited to, antibody fragments, monoclonal antibodies, bispecific antibodies, minibodies, domain antibodies, synthetic antibodies (sometimes referred to herein as "antibody mimetics"), chimeric antibodies, humanized antibodies, antibody fusions (sometimes referred to as "antibody conjugates"), and fragments of each, respectively. Structures that still rely

In one embodiment, the antibody is an antibody fragment. Specific antibody fragments include, but are not limited to, (i) the Fab fragment consisting of VL, VH, CL and CH1 domains, (ii) the Fd fragment consisting of the VH and CH1 domains, (iii) the Fv fragment consisting of the VL and VH domains of a single antibody; (iv) the dAb fragment (Ward et al., 1989, Nature 341:544-546, entirely incorporated by reference) which consists of a single variable, (v) isolated CDR regions, (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (Bird et al., 1988, Science 242:423-426, Huston et al., 1988, Proc. Natl. Acad. Sci. U.S.A. 85:5879-5883, entirely incorporated by reference), (viii) bispecific single chain Fv (WO 03/11161, hereby incorporated by reference) and (ix) "diabodies" or "triabodies", multivalent or multispecific fragments constructed by gene fusion (Tomlinson et. al., 2000, Methods Enzymol. 326:461-479; WO94/13804; Holliger et al., 1993, Proc. Natl. Acad. Sci. U.S.A. 90:6444-6448, all entirely incorporated by reference).

In some embodiments, the antibody can be a mixture from different species, e.g. a chimeric antibody and/or a humanized antibody. That is, in the present invention, the CDR sets can be used with framework and constant regions other than those specifically described by sequence herein.

In general, both "chimeric antibodies" and "humanized antibodies" refer to antibodies that combine regions from more than one species. For example, "chimeric antibodies" traditionally comprise variable region(s) from a mouse (or rat, in some cases) and the constant region(s) from a human. "Humanized antibodies" generally refer to non-human antibodies that have had the variable-domain framework regions swapped for sequences found in human antibodies. Generally, in a humanized antibody, the entire antibody, except the CDRs, is encoded by a polynucleotide of human origin or is identical to such an antibody except within its CDRs. The CDRs, some or all of which are encoded by nucleic acids originating in a non-human organism, are grafted into the beta-sheet framework of a human antibody variable region to create an antibody, the specificity of which is determined by the engrafted CDRs. The creation of such antibodies is described in, e.g., WO 92/11018, Jones, 1986, Nature 321:522-525, Verhoeyen et al., 1988, Science 239:1534-1536, all entirely incorporated by reference. "Backmutation" of selected acceptor framework residues to the corresponding donor residues is often required to regain affinity that is lost in the initial grafted construct (US 5530101; US 5585089; US 5693761; US 5693762; US 6180370; US 5859205; US 5821337; US 6054297; US 6407213, all entirely incorporated by reference). The humanized antibody optimally also will comprise at least a portion of an immunoglobulin constant region, typically that of a human immunoglobulin, and thus will typically comprise a human Fc region. Humanized antibodies can also be generated using mice with a genetically engineered immune system. Roque et al., 2004, Biotechnol. Prog. 20:639-654, entirely incorporated by reference. A variety of techniques and methods for humanizing and reshaping non-human antibodies are well known in the art (See Tsurushita & Vasquez, 2004, Humanization of Monoclonal Antibodies, Molecular Biology of B Cells, 533-545, Elsevier Science (USA), and references cited therein, all entirely incorporated by reference). Humanization methods include but are not limited to methods described in Jones et al., 1986, Nature 321:522-525; Riechmann et al.,1988; Nature 332:323-329; Verhoeyen et al., 1988, Science, 239:1534-1536; Queen et al., 1989, Proc Natl Acad Sci, USA 86:10029-33; He et al., 1998, J. Immunol. 160: 1029-1035; Carter et al., 1992, Proc Natl Acad Sci USA 89:4285-9, Presta et al., 1997, Cancer Res. 57(20):4593-9; Gorman et al., 1991, Proc. Natl. Acad. Sci. USA 88:4181-4185; O'Connor et al., 1998, Protein Eng 11:321-8, all entirely incorporated by reference. Humanization or other methods of reducing the immunogenicity of nonhuman antibody variable regions may include resurfacing methods, as described for example in Roguska et al., 1994, Proc. Natl. Acad. Sci. USA 91:969-973, entirely incorporated by reference. In one embodiment, the parent antibody has been affinity matured, as is known in the art. Structure-based methods may be employed for humanization and affinity maturation, for example as described in USSN 11/004,590. Selection based methods may be employed to humanize and/or affinity mature antibody variable regions, including but not limited to methods described in Wu et al., 1999, J. Mol. Biol. 294:151-162; Baca et al., 1997, J. Biol. Chem. 272(16):10678-10684; Rosok et al., 1996, J. Biol. Chem. 271(37): 22611-22618; Rader et al., 1998, Proc. Natl. Acad. Sci. USA 95: 8910-8915; Krauss et al., 2003, Protein Engineering 16(10):753-759, all entirely incorporated by reference. Other humanization methods may involve the grafting of only parts of the CDRs, including but not limited to methods described in USSN 09/810,510; Tan et al., 2002, J. Immunol. 169:1119-1125; De Pascalis et al., 2002, J. Immunol. 169:3076-3084, all entirely incorporated by reference.

In one embodiment, the antibodies of the invention can be multispecific antibodies, and notably bispecific antibodies. These are antibodies that bind to two (or more) different antigens, or different epitopes on the same antigen.

In some embodiments the antibodies are diabodies.

In one embodiment, the antibody is a minibody. Minibodies are minimized antibody-like proteins comprising a scFv joined to a CH3 domain. Hu et al., 1996, Cancer Res. 56:3055-3061, entirely incorporated by reference. In some cases, the scFv can be joined to the Fc region, and may include some or the entire hinge region.

The antibodies of the present invention are generally isolated or recombinant. An "isolated antibody," refers to an antibody which is substantially free of other antibodies having different antigenic specificities. For instance, an isolated antibody that specifically binds to EMP2 is substantially free of antibodies that specifically bind antigens other than EMP2.

An isolated antibody that specifically binds to an epitope, isoform or variant of human EMP2 or murine EMP2 may, however, have cross-reactivity to other related antigens, for instance from other species, such as EMP2 species homologs. Moreover, an isolated antibody may be substantially free of other cellular material and/or chemicals.

M A Q V Q L V Q S G G G V V Q P G R S L R L S C A A S G F T F S S Y
A M H W V R Q A P G K G L E W V A V I S Y D G S N K Y Y A D S V K G R F T I S R
D N S K N T L Y L Q M N S L R A E D T A V Y Y C A R D R R G R K S A G I D Y W G
Q G T L V T V S S

Isolated monoclonal antibodies, having different specificities, can be combined in a well defined composition. Thus, for example all possible combinations of the antibodies KS49, KS41, KS83, or KS89 can be combined in a single formulation, if desired.

The following human-origin antibody sequences encode for high-avidity antibodies specific for human (KS49, KS83) and mouse (KS83) EMP2 and have antibody variable region heavy and light chains suitable for use in either aspect of the invention:
KS49 heavy chain-
KS49 light chain- 
D I Q M T Q S P S S L S A S V G D R V T I T C Q A S Q D I S N Y L N W
Y Q Q K P G K A P K L L I Y A A S S L Q S G V P S R F S G S G S G T D F T L T I S S L
Q P E D F A T Y Y C L Q D Y N G W T F G Q G T K V D I K R A A A E Q K L I S E E D
L N G A A
KS83 heavy chain- 
M A Q V Q L V E S G G G L V Q P G G S L R L S C A A S G F T F S S Y
A M H W V R Q A P G K G L E W V A V I S Y D G S N K Y Y A D S V K G R F T I S R
D N S K N T L Y L Q M N S L R A E D T A V Y Y C A R T V G A T G A F D I W G Q G
T M V T V S S S
KS83 light chain-

D I V M T Q S P S T V S A S V G D R V I I P C R A S Q S I G K W L A W Y
Q Q K P G K A P K L L I Y K A S S L E G W V P S R F S G S G S G T E F S L T I S S L Q
P D D S A T Y V C Q Q S H N F P P T F G G G T K L E I K R A A A E Q K L I S E E D L
N G A A

Other diabodies for use according to either aspect of the invention include KS41 and KS89:
KS41 Heavy Chain- 
M A Q V Q L V Q S G G G L V Q P G R S L R L S C A A S G F S F S E Y P
M H W V R Q A P G R G L E S V A V I S Y D G E Y Q K Y A D S V K G R F T I S R D D
S K S T V Y L Q M N S L R P E D T A V Y Y C A R T I N N G M D V W G Q G T T V T
V S S
KS41 Light Chain- 
D I V M T Q S P S S L S A S V G D R V T I T C R A S Q G I R N D L G W
Y Q Q K P G K A P E L L I Y G A S S L Q S G V P S R F S G S G S G T D F T L T I S S L
Q P E D S A T Y Y C L Q D Y N G W T F G Q G T K L E I K R A A A E Q K L I S E E D
L N G A A
KS89 Heavy Chain- 
M A Q V Q L V Q S G G G L V Q P G R S L R L S C A A S G F S F S E Y P
M H W V R Q A P G R G L E S V A V I S Y D G E Y Q K Y A D S V K G R F T I S R D D
S K S T V Y L Q M N S L R P E D T A V Y Y C A R T I N N G M D V W G Q G T T V T
V S S
KS89 Light Chain-

D I V M T Q S P S S L S A S V G D R V T I T C R A S Q G I R N D L G W
Y Q Q K P G K A P E L L I Y G A S S L Q S G V P S R F S G S G S G T D F T L T I S S L
Q P E D S A T Y Y C L Q D Y N G W T F G Q G T K L E I K R A A A E Q K L I S E E D
L N G A A

Anti-EMP-2 variable region sequences, used to encode proteins on backbones including for native antibody, fragment antibody, or synthetic backbones, can avidly bind EMP-2. Via this binding, these proteins can be used for EMP-2 detection, and to block EMP-2 function. Expression of these variable region sequences on native antibody backbones, or as an scFv, triabody, diabody or minibody, labeled with radionuclide, are particularly useful in in the *in vivo* detection of EMP-2 bearing cells. Expression on these backbones or native antibody backbone are favorable for blocking the function of EMP-2 and/or killing EMP-2 bearing cells (e.g.gynecologic tumors) *in vivo.*

In some embodiments, the present invention provides anti-EMP-2 sequences comprising CDR regions of an antibody selected from KS49, KS83, KS41, and KS89, as shown in Figure 8. The CDR regions provided by the invention may be used to construct an anti-EMP-2 binding protein, including without limitation, an antibody, a scFv, a triabody, a diabody, a minibody, and the like. In a certain embodiment, an anti-EMP-2 binding protein of the invention will comprise at least one CDR region from an antibody selected from KS49, KS83, KS41, and KS89. Anti-EMP-2 binding proteins may comprise, for example, a CDR-H1, a CDR-H2, a CDR-H3, a CDR-L1, a CDR-L2, a CDR-L3, or combinations thereof, from an antibody provided herein. In particular embodiments of the invention, an anti-EMP-2 binding protein may comprise all three CDR-H sequences of an antibody provided herein, all three CDR-L sequences of an antibody provided herein, or both. Anti-EMP2 CDR sequences may be used on an antibody backbone, or fragment thereof, and likewise may include humanized antibodies, or antibodies containing humanized sequences. These antibodies may be used, for example, to detect EMP-2, to detect cells expressing EMP-2 *in vivo*, or to block EMP-2 function. In some embodiments, the CDR regions may be defined using the Kabat definition, the Chothia definition, the AbM definition, the contact definition, or any other suitable CDR numbering system.

In some embodiments, the CDRs are as follows:
CDR 1 Heavy
   SYAMH (49)
   SYAMH (83)
   EYPMH (41)
   EYPMH (89)
CDR 2 Heavy
VISYDGSNKYYADSVKG (49)
VISYDGSNKYYADSVKG (83)
VISYDGEYQKYADSVKG (41)
VISYDGEYQKYADSVKG (89)
CDR 1 Light
   QASQDISNYLN (49)
   RASQSIGKWLA (83)
   RASQGIRNDLG (41)
   RASQGIRNDLG (89)
CDR 2 Light
   AASSLQS (49)
   KASSLEG (83)
   GASSLQS (41)
   GASSLQS (89)
Diabody sequence (KS49)
Heavy chain, KS49 
M A Q V Q L V Q S G G G V V Q P G R S L R L S C A A S G F T F S S Y
A M H W V R Q A P G K G L E W V A V I S Y D G S N K Y Y A D S V K G R F T I S R
D N S K N T L Y L Q M N S L R A E D T A V Y Y C A R D R R G R K S A G I D Y W G
Q G T L V T V S
CDR1 SYAMH
[00147] CDR2 VISYDGSNKYYADSVKG
Light chain, KS49 
D I Q M T Q S P S S L S A S V G D R V T I T C Q A S Q D I S N Y L N W
Y Q Q K P G K A P K L L I Y A A S S L Q S G V P S R F S G S G S G T D F T L T I S S L
Q P E D F A T Y Y C L Q D Y N G W T F G Q G T K V D I K R A A A E Q K L I S E E D
L N G A A
[00150] CDR 1 QASQDISNYLN
CDR2 AASSLQS
Diabody sequence (KS83)
Heavy chain, KS83 
M A Q V Q L V E S G G G L V Q P G G S L R L S C A A S G F T F S S Y
A M H W V R Q A P G K G L E W V A V I S Y D G S N K Y Y A D S V K G R F T I S R
D N S K N T L Y L Q M N S L R A E D T A V Y Y C A R T V G A T G A F D I W G Q G
T M V T V S S
CDR1 SYAMH
CDR2 VISYDGSNKYYADSVKG
Light Chain, KS83 
D I V M T Q S P S T V S A S V G D R V I I P C R A S Q S I G K W L A W Y
Q Q K P G K A P K L L I Y K A S S L E G W V P S R F S G S G S G T E F S L T I S S L Q
P D D S A T Y V C Q Q S H N F P P T F G G G T K L E I K R A A A E Q K L I S E E D L
N G A A
CDR1 RASQSIGKWLA
CDR2 KASSLEG
Diabody sequence (KS41)
Heavy Chain, KS41 
M A Q V Q L V Q S G G G L V Q P G R S L R L S C A A S G F S F S E Y P
M H W V R Q A P G R G L E S V A V I S Y D G E Y Q K Y A D S V K G R F T I S R D D
S K S T V Y L Q M N S L R P E D T A V Y Y C A R T I N N G M D V W G Q G T T V T
V S S
CDR 1 EYPMH
CDR 2 VISYDGEYQKYADSVKG
Light Chain, KS41 
D I V M T Q S P S S L S A S V G D R V T I T C R A S Q G I R N D L G W
Y Q Q K P G K A P E L L I Y G A S S L Q S G V P S R F S G S G S G T D F T L T I S S L
Q P E D S A T Y Y C L Q D Y N G W T F G Q G T K L E I K R A A A E Q K L I S E E D
L N G A A
CDR 1 RASQGIRNDLG
CDR 2 GASSLQS
Diabody sequence (KS89)
Heavy Chain, KS89 
M A Q V Q L V Q S G G G L V Q P G R S L R L S C A A S G F S F S E Y P
Mt H W V R Q A P G R G L E S V A V I S Y D G E Y Q K Y A D S V K G R F T I S R D
D S K S T V Y L Q M N S L R P E D T A V Y Y C A R T I N N G M D V W G Q G T T V
T V S S
CDR1 EYPMH
CDR2 VISYDGEYQKYADSVKG
Light Chain, KS89 
D I V Met T Q S P S S L S A S V G D R V T I T C R A S Q G I R N D L G
W Y Q Q K P G K A P E L L I Y G A S S L Q S G V P S R F S G S G S G T D F T L T I S
S L Q P E D S A T Y Y C L Q D Y N G W T F G Q G T K L E I K R A A A E Q K L I S E
E D L N G A A
CDR 1 RASQGIRNDLG
CDR 2 GASSLQS

In some embodiments, the invention provides antibodies (e.g., diabodies, minibodies, triabodies) or fragments thereof having the CDRs of a diabody selected from KS49, KS83, KS41, and KS89. In some embodiments these antibodies lack the polyhistine tag. In other embodiments, the diabodies possess the light and heavy chain of a KS49, KS83, KS41, or KS89 diabody. In stillother embodiments, the antibodies are substantially identical in sequence to a diabody selected from the group consisting of KS49, KS83, KS41, and KS89 with or without the polyhistidine tag. In stillother embodiments, the antibodies are substantially identical in sequence to the light and heavy chain sequences of a diabody selected from the group consisting of KS49, KS83, KS41, and KS89. These identities can be 65%, 70%, 75%, 80%, 85%, 90%, and preferably 91%, 92%, 93%, 94%, 95%, 96%, 97%, 98% or 99% or greater amino acid sequence identity. In some further embodiments of any of the above, the antibodies comprise CDRs sequences identical to those of the KS49, KS83, KS41, or KS89 diabody.

The anti-EMP2 antibodies of the present invention specifically bind EMP2 ligands (e.g. the human and murine EMP2 proteins of SEQ ID NOs:1 and 2.

Specific binding for a particular antigen or an epitope can be exhibited, for example, by an antibody having a KD for an antigen or epitope of at least about 10⁻⁴ M, at least about 10⁻⁵ M, at least about 10⁻⁶ M, at least about 10⁻⁷ M, at least about 10⁻⁸ M, at least about 10⁻⁹ M, alternatively at least about 10⁻¹⁰ M, at least about 10⁻¹¹ M, at least about 10⁻¹² M, or greater, where KD refers to a dissociation rate of a particular antibody-antigen interaction. Typically, an antibody that specifically binds an antigen will have a KD that is 20-, 50-, 100-, 500-, 1000-, 5,000-, 10,000- or more times greater for a control molecule relative to the antigen or epitope.

Also, specific binding for a particular antigen or an epitope can be exhibited, for example, by an antibody having a KA or Ka for an antigen or epitope of at least 20-, 50-, 100-, 500-, 1000-, 5,000-, 10,000- or more times greater for the epitope relative to a control, where KA or Ka refers to an association rate of a particular antibody-antigen interaction.

The present invention further provides variant antibodies. That is, there are a number of modifications that can be made to the antibodies of the invention, including, but not limited to, amino acid modifications in the CDRs (affinity maturation), amino acid modifications in the Fc region, glycosylation variants, covalent modifications of other types, etc.

By "variant" herein is meant a polypeptide sequence that differs from that of a parent polypeptide by virtue of at least one amino acid modification. Amino acid modifications can include substitutions, insertions and deletions, with the former being preferred in many cases.

In general, variants can include any number of modifications, as long as the function of the protein is still present, as described herein. That is, in the case of amino acid variants generated with the CDRs of KS49, KS41, KS83, or KS89, for example, the antibody should still specifically bind to both human and/or murine EMP2. Similarly, if amino acid variants are generated with the Fc region, for example, the variant antibodies should maintain the required receptor binding functions for the particular application or indication of the antibody.

However, in general, from 1, 2, 3, 4, 5, 6, 7, 8, 9 or 10 amino acid substitutions are generally utilized as often the goal is to alter function with a minimal number of modifications. In some cases, there are from 1 to 5 modifications, with from 1-2, 1-3 and 1-4 also finding use in many embodiments.

It should be noted that the number of amino acid modifications may be within functional domains: for example, it may be desirable to have from 1-5 modifications in the Fc region of wild-type or engineered proteins, as well as from 1 to 5 modifications in the Fv region, for example. A variant polypeptide sequence will preferably possess at least about 80%, 85%, 90%, 95% or up to 98 or 99% identity to the parent sequences (e.g. the variable regions, the constant regions, and/or the heavy and light chain sequences for KS49, KS41, KS83, or KS89. It should be noted that depending on the size of the sequence, the percent identity will depend on the number of amino acids.

By "amino acid substitution" or "substitution" herein is meant the replacement of an amino acid at a particular position in a parent polypeptide sequence with another amino acid. For example, the substitution S100A refers to a variant polypeptide in which the serine at position 100 is replaced with alanine. By "amino acid insertion" or "insertion" as used herein is meant the addition of an amino acid at a particular position in a parent polypeptide sequence. By "amino acid deletion" or "deletion" as used herein is meant the removal of an amino acid at a particular position in a parent polypeptide sequence.

By "parent polypeptide", "parent protein", "precursor polypeptide", or "precursor protein" as used herein is meant an unmodified polypeptide that is subsequently modified to generate a variant. In general, the parent polypeptides herein are Ab79 and Ab19. Parent polypeptide may refer to the polypeptide itself, compositions that comprise the parent polypeptide, or the amino acid sequence that encodes it. Accordingly, by "parent Fc polypeptide" as used herein is meant an Fc polypeptide that is modified to generate a variant, and by "parent antibody" as used herein is meant an antibody that is modified to generate a variant antibody.

By "wild type" or "WT" or "native" herein is meant an amino acid sequence or a nucleotide sequence that is found in nature, including allelic variations. A WT protein, polypeptide, antibody, immunoglobulin, IgG, etc. has an amino acid sequence or a nucleotide sequence that has not been intentionally modified.

By "variant Fc region" herein is meant an Fc sequence that differs from that of a wild-type Fc sequence by virtue of at least one amino acid modification. Fc variant may refer to the Fc polypeptide itself, compositions comprising the Fc variant polypeptide, or the amino acid sequence.

In some embodiments, one or more amino acid modifications are made in one or more of the CDRs of the antibody (KS49, KS41, KS83, or KS89). In general, only 1 or 2 or 3amino acids are substituted in any single CDR, and generally no more than from 4, 5, 6, 7, 8 9 or 10 changes are made within a set of CDRs. However, it should be appreciated that any combination of no substitutions, 1, 2 or 3 substitutions in any CDR can be independently and optionally combined with any other substitution.

In some cases, amino acid modifications in the CDRs are referred to as "affinity maturation". An "affinity matured" antibody is one having one or more alteration(s) in one or more CDRs which results in an improvement in the affinity of the antibody for antigen, compared to a parent antibody which does not possess those alteration(s). In some cases, although rare, it may be desirable to decrease the affinity of an antibody to its antigen, but this is generally not preferred.

Affinity maturation can be done to increase the binding affinity of the antibody for the antigen by at least about 10% to 50-100-150% or more, or from 1 to 5 fold as compared to the "parent" antibody. Preferred affinity matured antibodies will have nanomolar or even picomolar affinities for the target antigen. Affinity matured antibodies are produced by known procedures. See, for example, Marks et al., 1992, Biotechnology 10:779-783 that describes affinity maturation by variable heavy chain (VH) and variable light chain (VL) domain shuffling. Random mutagenesis of CDR and/or framework residues is described in: Barbas, et al. 1994, Proc. Nat. Acad. Sci, USA 91:3809-3813; Shier et al., 1995, Gene 169:147-155; Yelton et al., 1995, J. Immunol. 155:1994-2004; Jackson et al., 1995, J. Immunol. 154(7):3310-9; and Hawkins et al, 1992, J. Mol. Biol. 226:889-896, for example.

Alternatively, amino acid modifications can be made in one or more of the CDRs of the antibodies of the invention that are "silent", e.g. that do not significantly alter the affinity of the antibody for the antigen. These can be made for a number of reasons, including optimizing expression (as can be done for the nucleic acids encoding the antibodies of the invention).

Thus, included within the definition of the CDRs and antibodies of the invention are variant CDRs and antibodies; that is, the antibodies of the invention can include amino acid modifications in one or more of the CDRs of KS49, KS41, KS83, or KS89. In addition, as outlined below, amino acid modifications can also independently and optionally be made in any region outside the CDRs, including framework and constant regions.

In some embodiments, the anti-EMP2 antibodies of the invention are composed of a variant Fc domain. As is known in the art, the Fc region of an antibody interacts with a number of Fc receptors and ligands, imparting an array of important functional capabilities referred to as effector functions. These Fc receptors include, but are not limited to, (in humans) FcγRI (CD64) including isoforms FcγRIa, FcγRIb, and FcγRIc; FcγRII (CD32), including isoforms FcγRIIa (including allotypes H131 and R131), FcγRIIb (including FcγRIIb-1 and FcγRIIb-2), and FcγRIIc; and FcγRIII (CD16), including isoforms FcγRIIIa (including allotypes V158 and F158, correlated to antibody-dependent cell cytotoxicity (ADCC)) and FcγRIIIb (including allotypes FcγRIIIb-NA1 and FcγRIIIb-NA2), FcRn (the neonatal receptor), C1q (complement protein involved in complement dependent cytotoxicity (CDC)) and FcRn (the neonatal receptor involved in serum half-life). Suitable modifications can be made at one or more positions as is generally outlined, for example in US Patent Application 11/841,654 and references cited therein, US 2004/013210, US 2005/0054832, US 2006/0024298, US 2006/0121032, US 2006/0235208, US 2007/0148170, USSN 12/341,769, US Patent No. 6,737,056, US Patent No. 7,670,600, US Patent No. 6,086,875 all of which are expressly incorporated by reference in their entirety, and in particular for specific amino acid substitutions that increase binding to Fc receptors.

In addition to the modifications outlined above, other modifications can be made. For example, the molecules may be stabilized by the incorporation of disulphide bridges linking the VH and VL domains (Reiter et al., 1996, Nature Biotech. 14:1239-1245, entirely incorporated by reference). In addition, there are a variety of covalent modifications of antibodies that can be made as outlined below.

Covalent modifications of antibodies are included within the scope of this invention, and are generally, but not always, done post-translationally. For example, several types of covalent modifications of the antibody are introduced into the molecule by reacting specific amino acid residues of the antibody with an organic derivatizing agent that is capable of reacting with selected side chains or the N- or C-terminal residues.

Cysteinyl residues most commonly are reacted with α-haloacetates (and corresponding amines), such as chloroacetic acid or chloroacetamide, to give carboxymethyl or carboxyamidomethyl derivatives. Cysteinyl residues may also be derivatized by reaction with bromotrifluoroacetone, α-bromo-β-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole and the like.

In addition, modifications at cysteines are particularly useful in antibody-drug conjugate (ADC) applications, further described below. In some embodiments, the constant region of the antibodies can be engineered to contain one or more cysteines that are particularly "thiol reactive", so as to allow more specific and controlled placement of the drug moiety. See for example US Patent No. 7,521,541, incorporated by reference in its entirety herein.

Histidyl residues are derivatized by reaction with diethylpyrocarbonate at pH 5.5-7.0 because this agent is relatively specific for the histidyl side chain. Para-bromophenacyl bromide also is useful; the reaction is preferably performed in 0.1M sodium cacodylate at pH 6.0.

Lysinyl and amino terminal residues are reacted with succinic or other carboxylic acid anhydrides. Derivatization with these agents has the effect of reversing the charge of the lysinyl residues. Other suitable reagents for derivatizing alpha-amino-containing residues include imidoesters such as methyl picolinimidate; pyridoxal phosphate; pyridoxal; chloroborohydride; trinitrobenzenesulfonic acid; O-methylisourea; 2,4-pentanedione; and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKa of the guanidine functional group. Furthermore, these reagents may react with the groups of lysine as well as the arginine epsilon-amino group.

The specific modification of tyrosyl residues may be made, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively. Tyrosyl residues are iodinated using 125I or 131I to prepare labeled proteins for use in radioimmunoassay, the chloramine T method described above being suitable.

Carboxyl side groups (aspartyl or glutamyl) are selectively modified by reaction with carbodiimides (R'-N=C=N--R'), where R and R' are optionally different alkyl groups, such as 1-cyclohexyl-3-(2-morpholinyl-4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Derivatization with bifunctional agents is useful for crosslinking antibodies to a water-insoluble support matrix or surface for use in a variety of methods, in addition to methods described below. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis (succinimidylpropionate), and bifunctional maleimides such as bis-N-maleimido-1,8-octane. Derivatizing agents such as methyl-3-[(p-azidophenyl)dithio]propioimidate yield photoactivatable intermediates that are capable of forming crosslinks in the presence of light. Alternatively, reactive water-insoluble matrices such as cynomolgusogen bromide-activated carbohydrates and the reactive substrates described in U.S. Pat. Nos. 3,969,287; 3,691,016; 4,195,128; 4,247,642; 4,229,537; and 4,330,440, all entirely incorporated by reference, are employed for protein immobilization.

Glutaminyl and asparaginyl residues are frequently deamidated to the corresponding glutamyl and aspartyl residues, respectively. Alternatively, these residues are deamidated under mildly acidic conditions. Either form of these residues falls within the scope of this invention.

Other modifications include hydroxylation of proline and lysine, phosphorylation of hydroxyl groups of seryl or threonyl residues, methylation of the α-amino groups of lysine, arginine, and histidine side chains (T. E. Creighton, Proteins: Structure and Molecular Properties, W. H. Freeman & Co., San Francisco, pp. 79-86 [1983], entirely incorporated by reference), acetylation of the N-terminal amine, and amidation of any C-terminal carboxyl group.

In addition, as will be appreciated by those in the art, labels (including fluorescent, enzymatic, magnetic, radioactive, etc. can all be added to the antibodies (as well as the other compositions of the invention).

Another type of covalent modification is alterations in glycosylation. In another embodiment, the antibodies disclosed herein can be modified to include one or more engineered glycoforms. By "engineered glycoform" as used herein is meant a carbohydrate composition that is covalently attached to the antibody, wherein said carbohydrate composition differs chemically from that of a parent antibody. Engineered glycoforms may be useful for a variety of purposes, including but not limited to enhancing or reducing effector function. A preferred form of engineered glycoform is afucosylation, which has been shown to be correlated to an increase in ADCC function, presumably through tighter binding to the FcγRIIIa receptor. In this context, "afucosylation" means that the majority of the antibody produced in the host cells is substantially devoid of fucose, e.g. 90-95-98% of the generated antibodies do not have appreciable fucose as a component of the carbohydrate moiety of the antibody (generally attached at N297 in the Fc region). Defined functionally, afucosylated antibodies generally exhibit at least a 50% or higher affinity to the FcγRIIIa receptor.

Engineered glycoforms may be generated by a variety of methods known in the art (Umaña et al., 1999, Nat Biotechnol 17:176-180; Davies et al., 2001, Biotechnol Bioeng 74:288-294; Shields et al., 2002, J Biol Chem 277:26733-26740; Shinkawa et al., 2003, J Biol Chem 278:3466-3473; US 6,602,684; USSN 10/277,370; USSN 10/113,929; PCT WO 00/61739A1; PCT WO 01/29246A1; PCT WO 02/31140A1; PCT WO 02/30954A1, all entirely incorporated by reference; (Potelligent® technology [Biowa, Inc., Princeton, NJ]; GlycoMAb® glycosylation engineering technology [Glycart Biotechnology AG, Zurich, Switzerland]). Many of these techniques are based on controlling the level of fucosylated and/or bisecting oligosaccharides that are covalently attached to the Fc region, for example by expressing an IgG in various organisms or cell lines, engineered or otherwise (for example Lec-13 CHO cells or rat hybridoma YB2/0 cells, by regulating enzymes involved in the glycosylation pathway (for example FUT8 [α1,6-fucosyltranserase] and/or β1-4-N-acetylglucosaminyltransferase III [GnTIII]), or by modifying carbohydrate(s) after the IgG has been expressed. For example, the "sugar engineered antibody" or "SEA technology" of Seattle Genetics functions by adding modified saccharides that inhibit fucosylation during production; see for example 20090317869, hereby incorporated by reference in its entirety. Engineered glycoform typically refers to the different carbohydrate or oligosaccharide; thus an antibody can include an engineered glycoform.

Alternatively, engineered glycoform may refer to the IgG variant that comprises the different carbohydrate or oligosaccharide. As is known in the art, glycosylation patterns can depend on both the sequence of the protein (e.g., the presence or absence of particular glycosylation amino acid residues, discussed below), or the host cell or organism in which the protein is produced. Particular expression systems are discussed below.

Glycosylation of polypeptides is typically either N-linked or O-linked. N-linked refers to the attachment of the carbohydrate moiety to the side chain of an asparagine residue. The tri-peptide sequences asparagine-X-serine and asparagine-X-threonine, where X is any amino acid except proline, are the recognition sequences for enzymatic attachment of the carbohydrate moiety to the asparagine side chain. Thus, the presence of either of these tri-peptide sequences in a polypeptide creates a potential glycosylation site. O-linked glycosylation refers to the attachment of one of the sugars N-acetylgalactosamine, galactose, or xylose, to a hydroxyamino acid, most commonly serine or threonine, although 5-hydroxyproline or 5-hydroxylysine may also be used.

Addition of glycosylation sites to the antibody is conveniently accomplished by altering the amino acid sequence such that it contains one or more of the above-described tri-peptide sequences (for N-linked glycosylation sites). The alteration may also be made by the addition of, or substitution by, one or more serine or threonine residues to the starting sequence (for O-linked glycosylation sites). For ease, the antibody amino acid sequence is preferably altered through changes at the DNA level, particularly by mutating the DNA encoding the target polypeptide at preselected bases such that codons are generated that will translate into the desired amino acids.

Another means of increasing the number of carbohydrate moieties on the antibody is by chemical or enzymatic coupling of glycosides to the protein. These procedures are advantageous in that they do not require production of the protein in a host cell that has glycosylation capabilities for N- and O-linked glycosylation. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330 and in Aplin and Wriston, 1981, CRC Crit. Rev. Biochem., pp. 259-306, both entirely incorporated by reference.

Removal of carbohydrate moieties present on the starting antibody (e.g. post-translationally) may be accomplished chemically or enzymatically. Chemical deglycosylation requires exposure of the protein to the compound trifluoromethanesulfonic acid, or an equivalent compound. This treatment results in the cleavage of most or all sugars except the linking sugar (N-acetylglucosamine or N-acetylgalactosamine), while leaving the polypeptide intact. Chemical deglycosylation is described by Hakimuddin et al., 1987, Arch. Biochem. Biophys. 259:52 and by Edge et al., 1981, Anal. Biochem. 118:131, both entirely incorporated by reference. Enzymatic cleavage of carbohydrate moieties on polypeptides can be achieved by the use of a variety of endo- and exo-glycosidases as described by Thotakura et al., 1987, Meth. Enzymol. 138:350, entirely incorporated by reference. Glycosylation at potential glycosylation sites may be prevented by the use of the compound tunicamycin as described by Duskin et al., 1982, J. Biol. Chem. 257:3105, entirely incorporated by reference. Tunicamycin blocks the formation of protein-N-glycoside linkages.

Another type of covalent modification of the antibody comprises linking the antibody to various nonproteinaceous polymers, including, but not limited to, various polyols such as polyethylene glycol, polypropylene glycol or polyoxyalkylenes, in the manner set forth in, for example, 2005-2006 PEG Catalog from Nektar Therapeutics (available at the Nektar website) US Patents 4,640,835; 4,496,689; 4,301,144; 4,670,417; 4,791,192 or 4,179,337, all entirely incorporated by reference. In addition, as is known in the art, amino acid substitutions may be made in various positions within the antibody to facilitate the addition of polymers such as PEG. See for example, U.S. Publication No. 2005/0114037A1, entirely incorporated by reference.

The present invention provides a number of antibodies each with a specific set of CDRs (including, as outlined above, some amino acid substitutions). As outlined above, the antibodies can be defined by sets of 6 CDRs, by variable regions, or by full-length heavy and light chains, including the constant regions. In addition, as outlined above, amino acid substitutions may also be made. In general, in the context of changes within CDRs, due to the relatively short length of the CDRs, the amino acid modifications are generally described in terms of the number of amino acid modifications that may be made. While this is also applicable to the discussion of the number of amino acid modifications that can be introduced in variable, constant or full length sequences, in addition to number of changes, it is also appropriate to define these changes in terms of the "% identity". Thus, as described herein, antibodies included within the invention are 80, 85, 90, 95, 98 or 99% identical to KS49, KS41, KS83, or KS89 described herein.

In some embodiments, antibodies that compete with the antibodies of the invention (for example, with KS49, KS41, KS83, or KS89) for binding to human EMP2 and/or murine EMP2 are provided. Competition for binding to EMP2 or a portion of EMP2 by two or more anti-EMP2 antibodies may be determined by any suitable technique, as is known in the art.

Competition in the context of the present invention refers to any detectably significant reduction in the propensity of an antibody of the invention (e.g. KS49, KS41, KS83, or KS89) to bind its particular binding partner, e.g. EMP2, in the presence of the test compound. Typically, competition means an at least about 10-100% reduction in the binding of an antibody of the invention to EMP2 in the presence of the competitor, as measured by standard techniques such as ELISA or Biacore® assays. Thus, for example, it is possible to set criteria for competitiveness wherein at least about 10% relative inhibition is detected; at least about 15% relative inhibition is detected; or at least about 20% relative inhibition is detected before an antibody is considered sufficiently competitive. In cases where epitopes belonging to competing antibodies are closely located in an antigen, competition may be marked by greater than about 40% relative inhibition of EMP2 binding (e.g., at least about 45% inhibition, such as at least about 50% inhibition, for instance at least about 55% inhibition, such as at least about 60% inhibition, for instance at least about 65% inhibition, such as at least about 70% inhibition, for instance at least about 75% inhibition, such as at least about 80% inhibition, for instance at least about 85% inhibition, such as at least about 90% inhibition, for instance at least about 95% inhibition, or higher level of relative inhibition).

In some cases, one or more of the components of the competitive binding assays are labeled.

It may also be the case that competition may exist between anti-EMP2 antibodies with respect to more than one of EMP2 epitope, and/or a portion of EMP2, e.g. in a context where the antibody-binding properties of a particular region of EMP2 are retained in fragments thereof, such as in the case of a well-presented linear epitope located in various tested fragments or a conformational epitope that is presented in sufficiently large EMP2 fragments as well as in EMP2.

Assessing competition typically involves an evaluation of relative inhibitory binding using an antibody of the invention, EMP2 (either human or murine or both), and the test molecule. Test molecules can include any molecule, including other antibodies, small molecules, peptides, etc. The compounds are mixed in amounts that are sufficient to make a comparison that imparts information about the selectivity and/or specificity of the molecules at issue with respect to the other present molecules.

The amounts of test compound, EMP2 and antibodies of the invention may be varied. For instance, for ELISA assessments about 5-50 µg (e.g., about 10-50 µg, about 20-50 µg, about 5-20 µg, about 10-20 µg, etc.) of the anti-EMP2 antibody and/or EMP2 targets are required to assess whether competition exists. Conditions also should be suitable for binding. Typically, physiological or near-physiological conditions (e.g., temperatures of about 20-40°C., pH of about 7-8, etc.) are suitable for anti-EMP2:EMP2 binding.

Often competition is marked by a significantly greater relative inhibition than about 5% as determined by ELISA and/or FACS analysis. It may be desirable to set a higher threshold of relative inhibition as a criteria/determinant of what is a suitable level of competition in a particular context (e.g., where the competition analysis is used to select or screen for new antibodies designed with the intended function of blocking the binding of another peptide or molecule binding to EMP2 (e.g., the natural binding partners of EMP2 or naturally occurring anti-EMP2 antibody).

In some embodiments, the anti-EMP2 antibody of the present invention specifically binds to one or more residues or regions in EMP2 but also does not cross-react with other proteins with homology to EMP2.

Typically, a lack of cross-reactivity means less than about 5% relative competitive inhibition between the molecules when assessed by ELISA and/or FACS analysis using sufficient amounts of the molecules under suitable assay conditions.

The disclosed antibodies may find use in blocking a ligand-receptor interaction or inhibiting receptor component interaction. The anti-EMP2 antibodies of the invention may be "blocking" or "neutralizing." A "neutralizing antibody" is intended to refer to an antibody whose binding to EMP2 results in inhibition of the biological activity of EMP2, for example its capacity to interact with ligands, enzymatic activity, and/or signaling capacity. Inhibition of the biological activity of EMP2 can be assessed by one or more of several standard in vitro or in vivo assays known in the art.

Inhibits binding" or "blocks binding" (for instance when referring to inhibition/blocking of binding of a EMP2 binding partner to EMP2) encompass both partial and complete inhibition/blocking. The inhibition/blocking of binding of a EMP2 binding partner to EMP2 may reduce or alter the normal level or type of cell signaling that occurs when a EMP2 binding partner binds to EMP2 without inhibition or blocking. Inhibition and blocking are also intended to include any measurable decrease in the binding affinity of a EMP2 binding partner to EMP2 when in contact with an anti-EMP2 antibody, as compared to the ligand not in contact with an anti-EMP2 antibody, for instance a blocking of binding of a EMP2 binding partner to EMP2 by at least about 10%, 20%, 30%, 40%, 50%, 60%, 70%, 80%, 90%, 99%, or 100%.

The present invention further provides methods for producing the disclosed anti-EMP2 antibodies. These methods encompass culturing a host cell containing isolated nucleic acid(s) encoding the antibodies of the invention. As will be appreciated by those in the art, this can be done in a variety of ways, depending on the nature of the antibody. In some embodiments, in the case where the antibodies of the invention are full length traditional antibodies, for example, a heavy chain variable region and a light chain variable region under conditions such that an antibody is produced and can be isolated.

In general, nucleic acids are provided that encode the antibodies of the invention. Such polynucleotides encode for both the variable and constant regions of each of the heavy and light chains, although other combinations are also contemplated by the present invention in accordance with the compositions described herein. The present invention also contemplates oligonucleotide fragments derived from the disclosed polynucleotides and nucleic acid sequences complementary to these polynucleotides.

The polynucleotides can be in the form of RNA or DNA. Polynucleotides in the form of DNA, cDNA, genomic DNA, nucleic acid analogs, and synthetic DNA are within the scope of the present invention. The DNA may be double-stranded or single-stranded, and if single stranded, may be the coding (sense) strand or non-coding (anti-sense) strand. The coding sequence that encodes the polypeptide may be identical to the coding sequence provided herein or may be a different coding sequence, which sequence, as a result of the redundancy or degeneracy of the genetic code, encodes the same polypeptides as the DNA provided herein.

In some embodiments, nucleic acid(s) encoding the antibodies of the invention are incorporated into expression vectors, which can be extrachromosomal or designed to integrate into the genome of the host cell into which it is introduced. Expression vectors can contain any number of appropriate regulatory sequences (including, but not limited to, transcriptional and translational control sequences, promoters, ribosomal binding sites, enhancers, origins of replication, etc.) or other components (selection genes, etc.), all of which are operably linked as is well known in the art. In some cases two nucleic acids are used and each put into a different expression vector (e.g. heavy chain in a first expression vector, light chain in a second expression vector), or alternatively they can be put in the same expression vector. It will be appreciated by those skilled in the art that the design of the expression vector(s), including the selection of regulatory sequences may depend on such factors as the choice of the host cell, the level of expression of protein desired, etc.

In general, the nucleic acids and/or expression can be introduced into a suitable host cell to create a recombinant host cell using any method appropriate to the host cell selected (e.g., transformation, transfection, electroporation, infection), such that the nucleic acid molecule(s) are operably linked to one or more expression control elements (e.g., in a vector, in a construct created by processes in the cell, integrated into the host cell genome). The resulting recombinant host cell can be maintained under conditions suitable for expression (e.g. in the presence of an inducer, in a suitable non-human animal, in suitable culture media supplemented with appropriate salts, growth factors, antibiotics, nutritional supplements, etc.), whereby the encoded polypeptide(s) are produced. In some cases, the heavy chains are produced in one cell and the light chain in another.

Mammalian cell lines available as hosts for expression are known in the art and include many immortalized cell lines available from the American Type Culture Collection (ATCC), Manassas, VA including but not limited to Chinese hamster ovary (CHO) cells, HEK 293 cells, NSO cells, HeLa cells, baby hamster kidney (BHK) cells, monkey kidney cells (COS), human hepatocellular carcinoma cells (e.g., Hep G2), and a number of other cell lines. Non-mammalian cells including but not limited to bacterial, yeast, insect, and plants can also be used to express recombinant antibodies. In some embodiments, the antibodies can be produced in transgenic animals such as cows or chickens.

### Nucleic Acids that interact with EMP2

Inhibitor Oligonucleotide and RNA interference (RNAi) Sequence Design. Known methods are used to identify sequences that inhibit candidate genes which are related to drug resistance and reduced survival rate. Such inhibitors may include but are not limited to, *siRNA* oligonucleotides, antisense oligonucleotides, peptide inhibitors and aptamer sequences that bind and act to inhibit PVT1 expression and/or function.

RNA interference is used to generate small double-stranded RNA (small interference RNA or *siRNA*) inhibitors to affect the expression of a candidate gene generally through cleaving and destroying its cognate RNA. Small interference RNA (*siRNA*) is typically 19-22 nt double-stranded RNA. *siRNA* can be obtained by chemical synthesis or by DNA-vector based RNAi technology. Using DNA vector based *siRNA* technology, a small DNA insert (about 70 bp) encoding a short hairpin RNA targeting the gene of interest is cloned into a commercially available vector. The insert-containing vector can be transfected into the cell, and expressing the short hairpin RNA. The hairpin RNA is rapidly processed by the cellular machinery into 19-22 nt double stranded RNA (*siRNA*)*.* In a preferred embodiment, the *siRNA* is inserted into a suitable RNAi vector because *siRNA* made synthetically tends to be less stable and not as effective in transfection.

*siRNA* can be made using methods and algorithms such as those described by Wang L, Mu F Y. (2004) A Web-based Design Center for Vector-based siRNA and siRNA cassette. Bioinformatics. (In press); Khvorova A, Reynolds A, Jayasena S D. (2003) Functional siRNAs and miRNAs exhibit strand bias. Cell. 115(2):209-16; Harborth J, Elbashir S M, Vandenburgh K, Manninga H, Scaringe S A, Weber K, Tuschl T. (2003) Sequence, chemical, and structural variation of small interfering RNAs and short hairpin RNAs and the effect on mammalian gene silencing. Antisense Nucleic Acid Drug Dev. 13(2):83-105; Reynolds A, Leake D, Boese Q, Scaringe S, Marshall W S, Khvorova A. (2004) Rational siRNA design for RNA interference. Nat Biotechnol. 22(3):326-30 and Ui-Tei K, Naito Y, Takahashi F, Haraguchi T, Ohki-Hamazaki H, Juni A, Ueda R, Saigo K. (2004) Guidelines for the selection of highly effective siRNA sequences for mammalian and chick RNA interference. Nucleic Acids Res. 32(3):936-48, which are hereby incorporated by reference.

Other tools for constructing *siRNA* sequences are web tools such as the *siRNA* Target Finder and Construct Builder available from GenScript (http://www.genscript.com), Oligo Design and Analysis Tools from Integrated DNA Technologies (URL:<http://www.idtdna.com/SciTools/SciTools.aspx>), or siDESIGN.TM. Center from Dharmacon, Inc. (URL:<http://design.dharmacon.com/default.aspx?source=0>). *siRNA* are suggested to built using the ORF (open reading frame) as the target selecting region, preferably 50-100 nt downstream of the start codon. Because siRNAs function at the mRNA level, not at the protein level, to design an *siRNA,* the precise target mRNA nucleotide sequence may be required. Due to the degenerate nature of the genetic code and codon bias, it is difficult to accurately predict the correct nucleotide sequence from the peptide sequence. Additionally, since the function of siRNAs is to cleave mRNA sequences, it is important to use the mRNA nucleotide sequence and not the genomic sequence for *siRNA* design, although as noted in the Examples, the genomic sequence can be successfully used for *siRNA* design. However, designs using genomic information might inadvertently target introns and as a result the *siRNA* would not be functional for silencing the corresponding mRNA.

Rational *siRNA* design should also minimize off-target effects which often arise from partial complementarity of the sense or antisense strands to an unintended target. These effects are known to have a concentration dependence and one way to minimize off-target effects is often by reducing *siRNA* concentrations. Another way to minimize such off-target effects is to screen the *siRNA* for target specificity.

The *siRNA* can be modified on the 5'-end of the sense strand to present compounds such as fluorescent dyes, chemical groups, or polar groups. Modification at the 5'-end of the antisense strand has been shown to interfere with *siRNA* silencing activity and therefore this position is not recommended for modification. Modifications at the other three termini have been shown to have minimal to no effect on silencing activity.

It is recommended that primers be designed to bracket one of the *siRNA* cleavage sites as this will help eliminate possible bias in the data (i.e., one of the primers should be upstream of the cleavage site, the other should be downstream of the cleavage site). Bias may be introduced into the experiment if the PCR amplifies either 5' or 3' of a cleavage site, in part because it is difficult to anticipate how long the cleaved mRNA product may persist prior to being degraded. If the amplified region contains the cleavage site, then no amplification can occur if the *siRNA* has performed its function.

Antisense oligonucleotides ("oligos") can be designed to inhibit candidate gene function. Antisense oligonucleotides are short single-stranded nucleic acids, which function by selectively hybridizing to their target mRNA, thereby blocking translation. Translation is inhibited by either RNase H nuclease activity at the DNA:RNA duplex, or by inhibiting ribosome progression, thereby inhibiting protein synthesis. This results in discontinued synthesis and subsequent loss of function of the protein for which the target mRNA encodes.

In a preferred embodiment, antisense oligos are phosphorothioated upon synthesis and purification, and are usually 18-22 bases in length. It is contemplated that the candidate gene antisense oligos may have other modifications such as 2'-O-Methyl RNA, methylphosphonates, chimeric oligos, modified bases and many others modifications, including fluorescent oligos.

In a preferred embodiment, active antisense oligos should be compared against control oligos that have the same general chemistry, base composition, and length as the antisense oligo. These can include inverse sequences, scrambled sequences, and sense sequences. The inverse and scrambled are recommended because they have the same base composition, thus same molecular weight and Tm as the active antisense oligonucleotides. Rational antisense oligo design should consider, for example, that the antisense oligos do not anneal to an unintended mRNA or do not contain motifs known to invoke immunostimulatory responses such as four contiguous G residues, palindromes of 6 or more bases and CG motifs.

Antisense oligonucleotides can be used in vitro in most cell types with good results. However, some cell types require the use of transfection reagents to effect efficient transport into cellular interiors. It is recommended that optimization experiments be performed by using differing final oligonucleotide concentrations in the 1-5 .mu.m range with in most cases the addition of transfection reagents. The window of opportunity, i.e., that concentration where you will obtain a reproducible antisense effect, may be quite narrow, where above that range you may experience confusing non-specific, non-antisense effects, and below that range you may not see any results at all. In a preferred embodiment, down regulation of the targeted mRNA will be demonstrated by use of techniques such as northern blot, real-time PCR, cDNA/oligo array or western blot. The same endpoints can be made for in vivo experiments, while also assessing behavioral endpoints.

For cell culture, antisense oligonucleotides should be re-suspended in sterile nuclease-free water (the use of DEPC-treated water is not recommended). Antisense oligonucleotides can be purified, lyophilized, and ready for use upon re-suspension. Upon suspension, antisense oligonucleotide stock solutions may be frozen at -20.degree. C. and stable for several weeks.

Aptamer sequences which bind to specific RNA or DNA sequences can be made. Aptamer sequences can be isolated through methods such as those disclosed in copending U.S. patent application Ser. No. 10/934,856, entitled, "Aptamers and Methods for their Invitro Selection and Uses Thereof," which is hereby incorporated by reference.

It is contemplated that the sequences described herein may be varied to result in substantially homologous sequences which retain the same function as the original. As used herein, a polynucleotide or fragment thereof is "substantially homologous" (or "substantially similar") to another if, when optimally aligned (with appropriate nucleotide insertions or deletions) with the other polynucleotide (or its complementary strand), using an alignment program such as BLASTN (Altschul, S. F., Gish, W., Miller, W., Myers, E. W. & Lipman, D. J. (1990) "Basic local alignment search tool." J. Mol. Biol. 215:403-410), and there is nucleotide sequence identity in at least about 80%, preferably at least about 90%, and more preferably at least about 95-98% of the nucleotide bases.

Mammalian cell lines available as hosts for expression are known in the art and include many immortalized cell lines available from the American Type Culture Collection (ATCC), Manassas, VA including but not limited to Chinese hamster ovary (CHO) cells, HEK 293 cells, NSO cells, HeLa cells, baby hamster kidney (BHK) cells, monkey kidney cells (COS), human hepatocellular carcinoma cells (e.g., Hep G2), and a number of other cell lines. Non-mammalian cells including but not limited to bacterial, yeast, insect, and plants can also be used to express recombinant antibodies. In some embodiments, the antibodies can be produced in transgenic animals such as cows or chickens.

### Methods of Treatment

### Antibody Compositions for In Vivo Administration

Formulations of the antibodies used in accordance with the present invention are prepared for storage by mixing an antibody having the desired degree of purity with optional pharmaceutically acceptable carriers, excipients or stabilizers (Remington's Pharmaceutical Sciences 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

The formulation herein may also contain more than one active compound as necessary for the particular indication being treated, preferably those with complementary activities that do not adversely affect each other. For example, it may be desirable to provide antibodies with other specificities. Alternatively, or in addition, the composition may comprise a cytotoxic agent, cytokine, growth inhibitory agent and/or small molecule antagonist. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for in vivo administration should be sterile, or nearly so. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and .gamma. ethyl-L-glutamate, nondegradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions.

### Administrative modalities

The antibodies and chemotherapeutic agents of the invention are administered to a subject, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Intravenous or subcutaneous administration of the antibody is preferred.

In certain aspects, the antibodies and chemotherapeutic agents of the invention are administered to a subject with cancer. In certain aspects, the antibodies and chemotherapeutic agents of the invention are administered to a subject with breast cancer. In certain aspects, the antibodies and chemotherapeutic agents of the invention are administered to a subject with triple negative breast cancer. In certain aspects, the antibodies and chemotherapeutic agents of the invention are administered to a subject with brain cancer, colon cancer, melanoma, leukemia (e.g., AML), pancreatic cancer, prostate cancer, ovarian cancer, lung cancer, and/or gastric cancer.

### Treatment modalities

In the methods of the invention, therapy is used to provide a positive therapeutic response with respect to a disease or condition. By "positive therapeutic response" is intended an improvement in the disease or condition, and/or an improvement in the symptoms associated with the disease or condition. For example, a positive therapeutic response would refer to one or more of the following improvements in the disease: (1) a reduction in the number of neoplastic cells; (2) an increase in neoplastic cell death; (3) inhibition of neoplastic cell survival; (5) inhibition (i.e., slowing to some extent, preferably halting) of tumor growth; (6) an increased patient survival rate; and (7) some relief from one or more symptoms associated with the disease or condition.

Positive therapeutic responses in any given disease or condition can be determined by standardized response criteria specific to that disease or condition. Tumor response can be assessed for changes in tumor morphology (i.e., overall tumor burden, tumor size, and the like) using screening techniques such as magnetic resonance imaging (MRI) scan, x-radiographic imaging, computed tomographic (CT) scan, bone scan imaging, endoscopy, and tumor biopsy sampling.

In addition to these positive therapeutic responses, the subject undergoing therapy may experience the beneficial effect of an improvement in the symptoms associated with the disease.

Such a response may persist for at least 4 to 8 weeks, or sometimes 6 to 8 weeks, following treatment according to the methods of the invention. Alternatively, an improvement in the disease may be categorized as being a partial response. By "partial response" is intended at least about a 50% decrease in all measurable tumor burden (i.e., the number of malignant cells present in the subject, or the measured bulk of tumor masses or the quantity of abnormal monoclonal protein) in the absence of new lesions, which may persist for 4 to 8 weeks, or 6 to 8 weeks.

Treatment according to the present invention includes a "therapeutically effective amount" of the medicaments used. A "therapeutically effective amount" refers to an amount effective, at dosages and for periods of time necessary, to achieve a desired therapeutic result.

A therapeutically effective amount may vary according to factors such as the disease state, age, sex, and weight of the individual, and the ability of the medicaments to elicit a desired response in the individual. A therapeutically effective amount is also one in which any toxic or detrimental effects of the antibody or antibody portion are outweighed by the therapeutically beneficial effects.

A "therapeutically effective amount" for tumor therapy may also be measured by its ability to stabilize the progression of disease. The ability of a compound to inhibit cancer may be evaluated in an animal model system predictive of efficacy in human tumors.

Alternatively, this property of a composition may be evaluated by examining the ability of the compound to inhibit cell growth or to induce apoptosis by in vitro assays known to the skilled practitioner. A therapeutically effective amount of a therapeutic compound may decrease tumor size, or otherwise ameliorate symptoms in a subject. One of ordinary skill in the art would be able to determine such amounts based on such factors as the subject's size, the severity of the subject's symptoms, and the particular composition or route of administration selected.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. Parenteral compositions may be formulated in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

The specification for the dosage unit forms of the present invention are dictated by and directly dependent on (a) the unique characteristics of the active compound and the particular therapeutic effect to be achieved, and (b) the limitations inherent in the art of compounding such an active compound for the treatment of sensitivity in individuals.

The efficient dosages and the dosage regimens for the anti-EMP2 antibodies used in the present invention depend on the disease or condition to be treated and may be determined by the persons skilled in the art.

An exemplary, non-limiting range for a therapeutically effective amount of an anti-EMP2 antibody used in the present invention is about 0.1-100 mg/kg, such as about 0.1-50 mg/kg, for example about 0.1-20 mg/kg, such as about 0.1-10 mg/kg, for instance about 0.5, about such as 0.3, about 1, or about 3 mg/kg. In another embodiment, he antibody is administered in a dose of 1 mg/kg or more, such as a dose of from 1 to 20 mg/kg, e.g. a dose of from 5 to 20 mg/kg, e.g. a dose of 8 mg/kg.

A medical professional having ordinary skill in the art may readily determine and prescribe the effective amount of the pharmaceutical composition required. For example, a physician or a veterinarian could start doses of the medicament employed in the pharmaceutical composition at levels lower than that required in order to achieve the desired therapeutic effect and gradually increase the dosage until the desired effect is achieved.

In one embodiment, the anti-EMP2 antibody is administered by infusion in a weekly dosage of from 10 to 500 mg/kg such as from 200 to 400 mg/kg. Such administration may be repeated, e.g., 1 to 8 times, such as 3 to 5 times. The administration may be performed by continuous infusion over a period of from 2 to 24 hours, such as from 2 to 12 hours.

In one embodiment, the anti-EMP2 antibody is administered by slow continuous infusion over a long period, such as more than 24 hours, if required to reduce side effects including toxicity.

In one embodiment the anti-EMP2 antibody is administered in a weekly dosage of from 250 mg to 2000 mg, such as for example 300 mg, 500 mg, 700 mg, 1000 mg, 1500 mg or 2000 mg, for up to 8 times, such as from 4 to 6 times. The administration may be performed by continuous infusion over a period of from 2 to 24 hours, such as from 2 to 12 hours. Such regimen may be repeated one or more times as necessary, for example, after 6 months or 12 months. The dosage may be determined or adjusted by measuring the amount of compound of the present invention in the blood upon administration by for instance taking out a biological sample and using anti-idiotypic antibodies which target the antigen binding region of the anti-EMP2 antibody.

In a further embodiment, the anti-EMP2 antibody is administered once weekly for 2 to 12 weeks, such as for 3 to 10 weeks, such as for 4 to 8 weeks.

In one embodiment, the anti-EMP2 antibody is administered by maintenance therapy, such as, e.g., once a week for a period of 6 months or more.

In one embodiment, the anti-EMP2 antibody is administered by a regimen including one infusion of an anti-EMP2 antibody followed by an infusion of an anti-EMP2 antibody conjugated to a radioisotope. The regimen may be repeated, e.g., 7 to 9 days later.

As non-limiting examples, treatment according to the present invention may be provided as a daily dosage of an antibody in an amount of about 0.1-100 mg/kg, such as 0.5, 0.9, 1.0, 1.1, 1.5, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 45, 50, 60, 70, 80, 90 or 100 mg/kg, per day, on at least one of day 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40, or alternatively, at least one of week 1, 2, 3, 4, 5, 6, 7, 8, 9, 10, 11, 12, 13, 14, 15, 16, 17, 18, 19 or 20 after initiation of treatment, or any combination thereof, using single or divided doses of every 24, 12, 8, 6, 4, or 2 hours, or any combination thereof.

### Combination Therapy

In some embodiments the anti-EMP2 antibody molecule thereof is used in combination with one or more additional therapeutic agents, e.g. a chemotherapeutic agent. Non-limiting examples of DNA damaging chemotherapeutic agents include topoisomerase I inhibitors (*e.g*., irinotecan, topotecan, camptothecin and analogs or metabolites thereof, and doxorubicin); topoisomerase II inhibitors (*e.g*., etoposide, teniposide, and daunorubicin); alkylating agents (*e.g*., melphalan, chlorambucil, busulfan, thiotepa, ifosfamide, carmustine, lomustine, semustine, streptozocin, decarbazine, methotrexate, mitomycin C, and cyclophosphamide); DNA intercalators (*e.g*., cisplatin, oxaliplatin, and carboplatin); DNA intercalators and free radical generators such as bleomycin; and nucleoside mimetics (*e.g*., 5-fluorouracil, capecitibine, gemcitabine, fludarabine, cytarabine, mercaptopurine, thioguanine, pentostatin, and hydroxyurea).

Chemotherapeutic agents that disrupt cell replication include: paclitaxel, docetaxel, and related analogs; vincristine, vinblastin, and related analogs; thalidomide, lenalidomide, and related analogs (*e.g*., CC-5013 and CC-4047); protein tyrosine kinase inhibitors (*e.g*., imatinib mesylate and gefitinib); proteasome inhibitors (*e.g*., bortezomib); NF-κB inhibitors, including inhibitors of IκB kinase; antibodies which bind to proteins overexpressed in cancers and other inhibitors of proteins or enzymes known to be upregulated, over-expressed or activated in cancers, the inhibition of which downregulates cell replication.

In some embodiments, the antibodies of the invention can be used prior to, concurrent with, or after treatment with any of the chemotherapeutic agents described herein or known to the skilled artisan at this time or subsequently.

### Efficacy of Methods Described Herein

In certain aspects of this invention, efficacy of anti-EMP2 therapy is measured by decreased serum concentrations of tumor specific markers, increased overall survival time, decreased tumor size, cancer remission, decreased metastasis marker response, and decreased chemotherapy adverse affects.

In certain aspects of this invention, efficacy is measured with companion diagnostic methods and products. Companion diagnostic measurements can be made before, during, or after anti-EMP2 treatment.

### Companion Diagnostics

In other embodiments, this disclsoure relates to companion diagnostic methods and products. In one embodiment, the companion diagnostic method and products can be used to monitor the regeneration and differentiation of CSCs. In one embodiment, the companion diagnostic method and products can be used to monitor the treatment of cancer. In a specific embodiment, the companion diagnostic method and products can be used to monitor the treatment of breast cancer. In a specific embodiment, the companion diagnostic method and products can be used to monitor the treatment of triple negative breast cancer. In one embodiment, the companion diagnostic method and products can be used to monitor the treatment of brain cancer, colon cancer, melanoma, leukemia (e.g., AML), pancreatic cancer, prostate cancer, ovarian cancer, lung cancer, and/or gastric cancer.

In some embodiments, the companion diagnostic methods and products include molecular assays to measure levels of proteins, genes or specific genetic mutations. Such measurements can be used, for example, to predict whether anti-EMP2 therapy will benefit a specific individual, to predict the effective dosage of anti-EMP2 therapy, to monitor anti-EMP2 therapy, adjust anti-EMP2 therapy, tailor the anti-EMP2 therapy to an individual, and track cancer progression and remission.

In some embodiments, the companion diagnostic can be used to monitor a combination therapy.

In some embodiments, the companion diagnostic can include an anti-EMP2 antibody described herein.

In some embodiments, the comapnion diagnostic can be used before, during, or after anti-EMP2 thearpy.

### Articles of Manufacture

In other embodiments, an article of manufacture containing materials useful for the treatment of the disorders described above is provided. The article of manufacture comprises a container and a label. Suitable containers include, for example, bottles, vials, syringes, and test tubes. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). The active agent in the composition is the antibody. The label on, or associated with, the container indicates that the composition is used for treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

### EXAMPLES

### Example 1. Anti-EMP2 Depletes Cancer Stem Cells in Tumors

In order to to exemplify the ability of anti-EMP2 antibody to deplete cancer stem cells in tumors, administered anti-EMP2 to a human triple negative breast cell line. The MDA-MB-231 human triple negative breast cell line was injected in nude mice to establish xenograft tumors, and the tumors were treated parenterally with either PG-101 (anti-EMP2 IgG1 monoclonal antibody) or control IgG1. The upper left line graph demonstrates the cessation of tumor growth observed with anti-EMP2 treatment. After treatment period, tumors were excised, and the frequency of tumor cells positive for the breast cancer stem cell marker aldefluor (an enzymatic assay for ALDH1) was assessed.

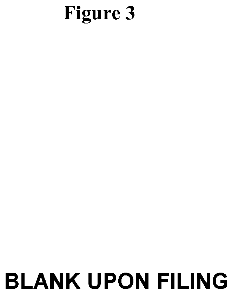

Anti-EMP2 treatment depleted biomarker-positive cancer stem cells (**Figure 3**, upper right bar graph).

### Example 2. Anti-EMP2 Treatment Prevents Reinitiation of Tumor Formation

A key feature of cancer stem cells is the capacity to reinitiate tumor formation in secondary hosts. To test whether anti-EMP2 antibody can prevent reinitiation of tumor formation, viable tumor cells isolated after treatment with anti-EMP2 IgG1 or control IgG1 were transferred into new recipient nude mice, and tested for the efficiency of tumor reinitiation. At limiting transferred cells (500, 5000, or 50000 cells), efficient reinitiation was observed with tumor cells after control IgG1 treatment, but minimal residual tumor reinitiation was observed with tumor cells after anti-EMP2 IgG1 treatment. These findings demonstrate that anti-EMP2 depletes cancer stem cells when enumerated by biomarkers or functionally assessed by tumor reinitiation.

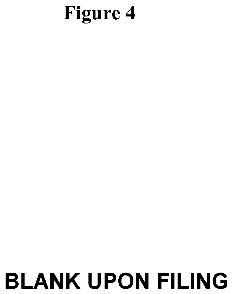

The equivalent effect of anti-EMP2 on cancer stem cells is also exemplified with HEC1A, a human endometrial cancer cell line (**Figure 4**). This demonstrates that the cancer stem cell effect of anti-EMP2 is displayed in human tumors of distinct tissue and epithelial origin.

### Example 3. Anti-EMP2 Treatment Prevents Reinitiation of Tumor Formation

To exemplify the action of anti-EMP2 antibody to deplete cancer stem cells in the context of combination chemotherapy, cancer stem cells were treated with a combination of docetaxel and anti-EMP2 antibody.

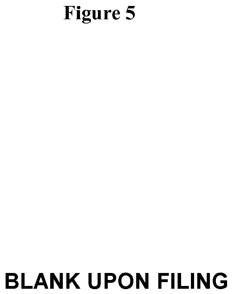

Cancer stem cells are resistant to and augmented in abundance by several categories of cytotoxic chemotherapy. This biology predicts that targeting cancer stem cells in the context of cytotoxic chemotherapy will be highly effective in tumor control. As described in **Figure 5**, MDA-MB-231 human breast cancer cells were inoculated in nude mice to establish xenografts, and then treated with a combination of docetaxel and anti-EMP2 IgG1 (bar indicates period of treatment). Tumor growth was slowed in the presence of either docetaxel or anti-EMP2 IgG1. However, in the presence of both agents, tumor receded, and in 60% of mice tumor was undetectable. This provides direct evidence of the predicted synergy of cancer chemotherapy (targeting bulk cancer cells) and anti-EMP2 (targeting cancer stem cells) for effective cancer therapy.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

## Claims

1. An antibody for use in the depletion of cancer stem cells expressing EMP2 in a patient having a cancer and previously identified as comprising cancer stem cells that express EMP2 and one or more markers selected from the group consisting of CD44, CD 133 ABCG2, and ALDH, wherein:
a) the antibody comprises a heavy chain having the amino acid sequence:
MAQVQLVQSGGGVVQPGRSLRLSCAASGFTFSSYAMHWVRQAPGKGLEWVAVISYDGSNKYYADSVKGRFTISRDNSKNTLYLQMNSLRAEDTAVYYCARDRRGRKSAGIDYWGQGTLVTVSS, and a light chain having the amino acid sequence: 
DIQMTQSPSSLSASVGDRVTITCQASQDISNYLNWYQQKPGKAPKLLIYAASSLQ
SGVPSRFSGSGSGTDFTLTISSLQPEDFATYYCLQDYNGWTFGQGTKVDIKRAAA
EQKLISEEDLNGAA;
b) the antibody comprises a heavy chain having the amino acid sequence:
MAQVQLVQSGGGLVQPGRSLRLSCAASGFSFSEYPMHWVRQAPGRGLESVAVISYDGEYQKYADSVKGRFTISRDDSKSTVYLQMNSLRPEDTAVYYCARTINNGMDVWGQGTTVT, and a light chain having the amino acid sequence: 
DIVMTQSPSSLSASVGDRVTITCRASQGIRNDLGWYQQKPGKAPELLIYGASSLQ
SGVPSRFSGSGSGTDFTLTISSLQPEDSATYYCLQDYNGWTFGQGTKLEIKRAAA
EQKLISEEDLNGAA;
c) the antibody comprises a heavy chain having the amino acid sequence:
MAQVQLVESGGGLVQPGGSLRLSCAASGFTFSSYAMHWVRQAPGKGLEWVAVISYDGSNKYYADSVKGRFTISRDNSKNTLYLQMNSLRAEDTAVYYCARTVGATGAFDIWGQGTMVTVSSS, and a light chain having the amino acid sequence: 
DIVMTQSPSTVSASVGDRVIIPCRASQSIGKWLAWYQQKPGKAPKLLIYKASSLE
GWVPSRFSGSGSGTEFSLTISSLQPDDSATYVCQQSHNFPPTFGGGTKLEIKRAAA
EQKLISEEDLNGAA;
d) the antibody comprises a heavy chain having the amino acid sequence:
MAQVQLVQSGGGLVQPGRSLRLSCAASGFSFSEYPMHWVRQAPGRGLESVAVISYDGEYQKYADSVKGRFTISRDDSKSTVYLQMNSLRPEDTAVYYCARTINNGMDVWGQGTTVT, and a light chain having the amino acid sequence: 
DIVMTQSPSSLSASVGDRVTITCRASQGIRNDLGWYQQKPGKAPELLIYGASSLQ
SGVPSRFSGSGSGTDFTLTISSLQPEDSATYYCLQDYNGWTFGQGTKLEIKRAAA
EQKLISEEDLNGAA;
e) the antibody competes for binding to EMP2 with an antibody according to a), b), c), or d);
f) the antibody shares 90% amino acid identity with heavy and light chain variable regions of an antibody according to a), b), c), or d); or
g) the antibody comprises CDR sequences identical to those of an antibody according to a), b), c), or d).

2. The antibody for use according to claim 1, wherein said use further comprises administration of at least one additional anti-cancer agent.

3. The antibody for use according to claim 2, wherein the at least one additional anti-cancer agent
(i) is selected from the group consisting of platinum-based chemotherapy drugs, taxanes, tyrosine kinase inhibitors, anti-EGFR antibodies, anti-ErbB2 antibodies, and combinations thereof,
(ii) comprises an EGFR inhibitor, or
(iii) comprises a VEGF inhibitor.

4. The antibody for use according to any of claims 1-3 wherein the antibody is conjugated with an effector moiety, for example a toxic agent.

5. The antibody for use according to any of claims 1-4, wherein the cancer is breast cancer.

6. The antibody for use according to any one of claims 1-4, wherein the cancer is a cancer selected from a group comprising brain cancer, colon cancer, melanoma, leukemia (*e.g.,* AML), pancreatic cancer, prostate cancer, ovarian cancer, lung cancer, and gastric cancer.

7. The antibody for use according to any one of claims 1-6, wherein said use further comprises use of a companion diagnostic, for example an anti-EMP2 antibody.

8. The antibody for use according to any of claims 1-3, wherein the cancer is endometrial cancer.

9. An in vitro method of detecting cancer stem cells, the method comprising:
providing a biological sample derived from a human having or suspected of having cancer; and detecting the expression of EMP2 and one or more markers selected from the group consisting of CD44, CD 133, ABCG2, and ALDH, wherein the EMP2 expression is detected with an antibody which shares 90% amino acid identity with heavy and light chain variable regions of a diabody comprising:
a) a heavy chain having the amino acid sequence:
MAQVQLVQSGGGVVQPGRSLRLSCAASGFTFSSYAMHWVRQAPGKGLEWVAVISYDGSNKYYADSVKGRFTISRDNSKNTLYLQMNSLRAEDTAVYYCARDRRGRKSAGIDYWGQGTLVTVSS, and a light chain having the amino acid sequence: 
DIQMTQSPSSLSASVGDRVTITCQASQDISNYLNWYQQKPGKAPKLLIYAASSLQ
SGVPSRFSGSGSGTDFTLTISSLQPEDFATYYCLQDYNGWTFGQGTKVDIKRAAA
EQKLISEEDLNGAA;
b) a heavy chain having the amino acid sequence:
MAQVQLVQSGGGLVQPGRSLRLSCAASGFSFSEYPMHWVRQAPGRGLESVAVISYDGEYQKYADSVKGRFTISRDDSKSTVYLQMNSLRPEDTAVYYCARTINNGMDVWGQGTTVT, and a light chain having the amino acid sequence: 
DIVMTQSPSSLSASVGDRVTITCRASQGIRNDLGWYQQKPGKAPELLIYGASSLQ
SGVPSRFSGSGSGTDFTLTISSLQPEDSATYYCLQDYNGWTFGQGTKLEIKRAAA
EQKLISEEDLNGAA;
c) a heavy chain having the amino acid sequence:
MAQVQLVESGGGLVQPGGSLRLSCAASGFTFSSYAMHWVRQAPGKGLEWVAVISYDGSNKYYADSVKGRFTISRDNSKNTLYLQMNSLRAEDTAVYYCARTVGATGAFDIWGQGTMVTVSSS, and a light chain having the amino acid sequence: 
DIVMTQSPSTVSASVGDRVIIPCRASQSIGKWLAWYQQKPGKAPKLLIYKASSLE
GWVPSRFSGSGSGTEFSLTISSLQPDDSATYVCQQSHNFPPTFGGGTKLEIKRAAA
EQKLISEEDLNGAA; or
d) a heavy chain having the amino acid sequence:
MAQVQLVQSGGGLVQPGRSLRLSCAASGFSFSEYPMHWVRQAPGRGLESVAVISYDGEYQKYADSVKGRFTISRDDSKSTVYLQMNSLRPEDTAVYYCARTINNGMDVWGQGTTVT, and a light chain having the amino acid sequence:

DIVMTQSPSSLSASVGDRVTITCRASQGIRNDLGWYQQKPGKAPELLIYGASSLQ
SGVPSRFSGSGSGTDFTLTISSLQPEDSATYYCLQDYNGWTFGQGTKLEIKRAAA
EQKLISEEDLNGAA.

10. The method of claim 9, wherein the EMP2 expression is detected with an antibody comprising:
a) a heavy chain having the amino acid sequence:
MAQVQLVQSGGGWQPGRSLRLSCAASGFTFSSYAMHWVRQAPGKGLEWVAVISYDGSNKYYADSVKGRFTISRDNSKNTLYLQMNSLRAEDTAVYYCARDRRGRKSAGIDYWGQGTLVTVSS, and a light chain having the amino acid sequence: 
DIQMTQSPSSLSASVGDRVTITCQASQDISNYLNWYQQKPGKAPKLLIYAASSLQ
SGVPSRFSGSGSGTDFTLTISSLQPEDFATYYCLQDYNGWTFGQGTKVDIKRAAA
EQKLISEEDLNGAA;
b) a heavy chain having the amino acid sequence:
MAQVQLVQSGGGLVQPGRSLRLSCAASGFSFSEYPMHWVRQAPGRGLESVAVISYDGEYQKYADSVKGRFTISRDDSKSTVYLQMNSLRPEDTAVYYCARTINNGMDVWGQGTTVT, and a light chain having the amino acid sequence: 
DIVMTQSPSSLSASVGDRVTITCRASQGIRNDLGWYQQKPGKAPELLIYGASSLQ
SGVPSRFSGSGSGTDFTLTISSLQPEDSATYYCLQDYNGWTFGQGTKLEIKRAAA
EQKLISEEDLNGAA;
c) a heavy chain having the amino acid sequence:
MAQVQLVESGGGLVQPGGSLRLSCAASGFTFSSYAMHWVRQAPGKGLEWVAVISYDGSNKYYADSVKGRFTISRDNSKNTLYLQMNSLRAEDTAVYYCARTVGATGAFDIWGQGTMVTVSSS, and a light chain having the amino acid sequence: 
DIVMTQSPSTVSASVGDRVIIPCRASQSIGKWLAWYQQKPGKAPKLLIYKASSLE
GWVPSRFSGSGSGTEFSLTISSLQPDDSATYVCQQSHNFPPTFGGGTKLEIKRAAA
EQKLISEEDLNGAA; or
d) a heavy chain having the amino acid sequence:
MAQVQLVQSGGGLVQPGRSLRLSCAASGFSFSEYPMHWVRQAPGRGLESVAVISYDGEYQKYADSVKGRFTISRDDSKSTVYLQMNSLRPEDTAVYYCARTINNGMDVWGQGTTVT, and a light chain having the amino acid sequence:

DIVMTQSPSSLSASVGDRVTITCRASQGIRNDLGWYQQKPGKAPELLIYGASSLQ
SGVPSRFSGSGSGTDFTLTISSLQPEDSATYYCLQDYNGWTFGQGTKLEIKRAAA
EQKLISEEDLNGAA.

11. The method of claim 9 or 10, wherein the human has or is suspected of having breast cancer, for example triple negative breast cancer, or endometrial cancer.

## Patentansprüche

1. Ein Antikörper zur Verwendung bei der Depletion von EMP2 exprimierenden Krebsstammzellen bei einem Patienten, der einen Krebs hat und der zuvor als Krebsstammzellen, die EMP2 und einen oder mehrere aus der Gruppe, bestehend aus CD44, CD133, ABCG2 und ALDH, ausgewählte Marker exprimieren, beinhaltend identifiziert wurde, wobei:
a) der Antikörper eine schwere Kette mit der Aminosäuresequenz:
MAQVQLVQSGGGVVQPGRSLRLSCAASGFTFSSYAMHWVRQAPGKGLEWVAVISYDGSNKYYADSVKGRFTISRDNSKNTLYLQMNSLRAEDTAVYYCARDRRGRKSAGIDYWGQGTLVTVSS und eine leichte Kette mit der Aminosäuresequenz: DIQMTQSPSSLSASVGDRVTITCQASQDISNYLNWYQQKPGKAPKLLIYAASSLQSGVPSRFSGSGSGTDFTLTISSLQPEDFATYYCLQDYNGWTFGQGTKVDIKRAAAEQKLISEEDLNGAA beinhaltet;
b) der Antikörper eine schwere Kette mit der Aminosäuresequenz:
MAQVQLVQSGGGLVQPGRSLRLSCAASGFSFSEYPMHWVRQAPGRGLESVAVISYDGEYQKYADSVKGRFTISRDDSKSTVYLQMNSLRPEDTAVYYCARTINNGMDVWGQGTTVT und eine leichte Kette mit der Aminosäuresequenz:
DIVMTQSPSSLSASVGDRVTITCRASQGIRNDLGWYQQKPGKAPELLIYGASSLQSGVPSRFSGSGSGTDFTLTISSLQPEDSATYYCLQDYNGWTFGQGTKLEIKRAAAEQKLISEEDLNGAA beinhaltet;
c) der Antikörper eine schwere Kette mit der Aminosäuresequenz:
MAQVQLVESGGGLVQPGGSLRLSCAASGFTFSSYAMHWVRQAPGKGLEWVAVISYDGSNKYYADSVKGRFTISRDNSKNTLYLQMNSLRAEDTAVYYCARTVGATGAFDIWGQGTMVTVSSS und eine leichte Kette mit der Aminosäuresequenz:
DIVMTQSPSTVSASVGDRVIIPCRASQSIGKWLAWYQQKPGKAPKLLIYKASSLEGWVPSRFSGSGSGTEFSLTISSLQPDDSATYVCQQSHNFPPTFGGGTKLEIKRAAAEQKLISEEDLNGAA beinhaltet;
d) der Antikörper eine schwere Kette mit der Aminosäuresequenz:
MAQVQLVQSGGGLVQPGRSLRLSCAASGFSFSEYPMHWVRQAPGRGLESVAVISYDGEYQKYADSVKGRFTISRDDSKSTVYLQMNSLRPEDTAVYYCARTINNGMDVWGQGTTVT und eine leichte Kette mit der Aminosäuresequenz:
DIVMTQSPSSLSASVGDRVTITCRASQGIRNDLGWYQQKPGKAPELLIYGASSLQSGVPSRFSGSGSGTDFTLTISSLQPEDSATYYCLQDYNGWTFGQGTKLEIKRAAAEQKLISEEDLNGAA beinhaltet;
e) der Antikörper mit einem Antikörper gemäß a), b), c) oder d) um die Bindung an EMP2 konkurriert;
f) der Antikörper 90%ige Aminosäureidentität mit variablen Regionen der schweren und leichten Ketten eines Antikörpers gemäß a), b), c) oder d) teilt; oder
g) der Antikörper CDR-Sequenzen beinhaltet, die mit denen eines Antikörpers gemäß a), b), c) oder d) identisch sind.

2. Antikörper zur Verwendung gemäß Anspruch 1, wobei die Verwendung ferner die Verabreichung von mindestens einem zusätzlichen Antikrebsmittel beinhaltet.

3. Antikörper zur Verwendung gemäß Anspruch 2, wobei das mindestens eine zusätzliche Antikrebsmittel
(i) aus der Gruppe, bestehend aus Chemotherapeutika auf Platinbasis, Taxanen, Tyrosinkinase-Inhibitoren, Anti-EGFR-Antikörpern, Anti-ErbB2-Antikörpern und Kombinationen davon, ausgewählt ist,
(ii) einen EGFR-Inhibitor beinhaltet oder
(iii) einen VEGF-Inhibitor beinhaltet.

4. Antikörper zur Verwendung gemäß einem der Ansprüche 1-3, wobei der Antikörper mit einer Effektoreinheit, zum Beispiel einem toxischen Stoff, konjugiert ist.

5. Antikörper zur Verwendung gemäß einem der Ansprüche 1-4, wobei der Krebs Brustkrebs ist.

6. Antikörper zur Verwendung gemäß einem der Ansprüche 1-4, wobei der Krebs ein Krebs, ausgewählt aus einer Gruppe, beinhaltend Gehirnkrebs, Darmkrebs, Melanom, Leukämie (z. B. AML), Bauchspeicheldrüsenkrebs, Prostatakrebs, Eierstockkrebs, Lungenkrebs und Magenkrebs, ist.

7. Antikörper zur Verwendung gemäß einem der Ansprüche 1-6, wobei die Verwendung ferner die Verwendung eines Begleitdiagnostikums, zum Beispiel eines Anti-EMP2-Antikörpers, beinhaltet.

8. Antikörper zur Verwendung gemäß einem der Ansprüche 1-3, wobei der Krebs Endometriumkrebs ist.

9. Ein In-vitro-Verfahren zum Nachweis von Krebsstammzellen, wobei das Verfahren Folgendes beinhaltet: Bereitstellen einer biologischen Probe, die von einem Menschen stammt, der Krebs hat oder vermutlich Krebs hat; und Nachweisen der Expression von EMP2 und einem oder mehreren Markern, die aus der Gruppe, bestehend aus CD44, CD133, ABCG2 und ALDH, ausgewählt sind, wobei die EMP2-Expression mit einem Antikörper nachgewiesen wird, der 90%ige Aminosäureidentität mit variablen Regionen der schweren und leichten Ketten eines Diakörpers teilt, beinhaltend:
a) eine schwere Kette mit der Aminosäuresequenz:
MAQVQLVQSGGGVVQPGRSLRLSCAASGFTFSSYAMHWVRQAPGKGLEWVAVISYDGSNKYYADSVKGRFTISRDNSKNTLYLQMNSLRAEDTAVYYCARDRRGRKSAGIDYWGQGTLVTVSS und eine leichte Kette mit der Aminosäuresequenz: 
DIQMTQSPSSLSASVGDRVTITCQASQDISNYLNWYQQKPGKAPKLLIYAASSLQ
SGVPSRFSGSGSGTDFTLTISSLQPEDFATYYCLQDYNGWTFGQGTKVDIKRAAA
EQKLISEEDLNGAA;
b) eine schwere Kette mit der Aminosäuresequenz:
MAQVQLVQSGGGLVQPGRSLRLSCAASGFSFSEYPMHWVRQAPGRGLESVAVISYDGEYQKYADSVKGRFTISRDDSKSTVYLQMNSLRPEDTAVYYCARTINNGMDVWGQGTTVT und eine leichte Kette mit der Aminosäuresequenz: 
DIVMTQSPSSLSASVGDRVTITCRASQGIRNDLGWYQQKPGKAPELLIYGASSLQ
SGVPSRFSGSGSGTDFTLTISSLQPEDSATYYCLQDYNGWTFGQGTKLEIKRAAA
EQKLISEEDLNGAA;
c) eine schwere Kette mit der Aminosäuresequenz:
MAQVQLVESGGGLVQPGGSLRLSCAASGFTFSSYAMHWVRQAPGKGLEWVAVISYDGSNKYYADSVKGRFTISRDNSKNTLYLQMNSLRAEDTAVYYCARTVGATGAFDIWGQGTMVTVSSS und eine leichte Kette mit der Aminosäuresequenz: 
DIVMTQSPSTVSASVGDRVIIPCRASQSIGKWLAWYQQKPGKAPKLLIYKASSLE
GWVPSRFSGSGSGTEFSLTISSLQPDDSATYVCQQSHNFPPTFGGGTKLEIKRAAA
EQKLISEEDLNGAA; oder
d) eine schwere Kette mit der Aminosäuresequenz:
MAQVQLVQSGGGLVQPGRSLRLSCAASGFSFSEYPMHWVRQAPGRGLESVAVISYDGEYQKYADSVKGRFTISRDDSKSTVYLQMNSLRPEDTAVYYCARTINNGMDVWGQGTTVT und eine leichte Kette mit der Aminosäuresequenz:

DIVMTQSPSSLSASVGDRVTITCRASQGIRNDLGWYQQKPGKAPELLIYGASSLQ
SGVPSRFSGSGSGTDFTLTISSLQPEDSATYYCLQDYNGWTFGQGTKLEIKRAAA
EQKLISEEDLNGAA.

10. Verfahren gemäß Anspruch 9, wobei die EMP2-Expression mit einem Antikörper nachgewiesen wird, der Folgendes beinhaltet:
a) eine schwere Kette mit der Aminosäuresequenz:
MAQVQLVQSGGGVVQPGRSLRLSCAASGFTFSSYAMHWVRQAPGKGLEWVAVISYDGSNKYYADSVKGRFTISRDNSKNTLYLQMNSLRAEDTAVYYCARDRRGRKSAGIDYWGQGTLVTVSS und eine leichte Kette mit der Aminosäuresequenz: DIQMTQSPSSLSASVGDRVTITCQASQDISNYLNWYQQKPGKAPKLLIYAASSLQ 
SGVPSRFSGSGSGTDFTLTISSLQPEDFATYYCLQDYNGWTFGQGTKVDIKRAAA
EQKLISEEDLNGAA;
b) eine schwere Kette mit der Aminosäuresequenz:
MAQVQLVQSGGGLVQPGRSLRLSCAASGFSFSEYPMHWVRQAPGRGLESVAVISYDGEYQKYADSVKGRFTISRDDSKSTVYLQMNSLRPEDTAVYYCARTINNGMDVWGQGTTVT und eine leichte Kette mit der Aminosäuresequenz: 
DIVMTQSPSSLSASVGDRVTITCRASQGIRNDLGWYQQKPGKAPELLIYGASSLQ
SGVPSRFSGSGSGTDFTLTISSLQPEDSATYYCLQDYNGWTFGQGTKLEIKRAAA
EQKLISEEDLNGAA;
c) eine schwere Kette mit der Aminosäuresequenz:
MAQVQLVESGGGLVQPGGSLRLSCAASGFTFSSYAMHWVRQAPGKGLEWVAVISYDGSNKYYADSVKGRFTISRDNSKNTLYLQMNSLRAEDTAVYYCARTVGATGAFDIWGQGTMVTVSSS und eine leichte Kette mit der Aminosäuresequenz: 
DIVMTQSPSTVSASVGDRVIIPCRASQSIGKWLAWYQQKPGKAPKLLIYKASSLE
GWVPSRFSGSGSGTEFSLTISSLQPDDSATYVCQQSHNFPPTFGGGTKLEIKRAAA
EQKLISEEDLNGAA; oder
d) eine schwere Kette mit der Aminosäuresequenz:
MAQVQLVQSGGGLVQPGRSLRLSCAASGFSFSEYPMHWVRQAPGRGLESVAVISYDGEYQKYADSVKGRFTISRDDSKSTVYLQMNSLRPEDTAVYYCARTINNGMDVWGQGTTVT und eine leichte Kette mit der Aminosäuresequenz:

DIVMTQSPSSLSASVGDRVTITCRASQGIRNDLGWYQQKPGKAPELLIYGASSLQ
SGVPSRFSGSGSGTDFTLTISSLQPEDSATYYCLQDYNGWTFGQGTKLEIKRAAA
EQKLISEEDLNGAA.

11. Verfahren gemäß Anspruch 9 oder 10, wobei der Mensch Brustkrebs, zum Beispiel triple-negativen Brustkrebs, oder Endometriumkrebs hat oder vermutlich hat.

## Revendications

1. Un anticorps pour une utilisation dans la déplétion de cellules souches cancéreuses exprimant EMP2 chez un patient ayant un cancer et identifié précédemment comme comprenant des cellules souches cancéreuses qui expriment EMP2 et un ou plusieurs marqueurs sélectionnés dans le groupe constitué de CD44, CD133, ABCG2 et ALDH :
a) l'anticorps comprenant une chaîne lourde ayant la séquence d'acides aminés :
MAQVQLVQSGGGVVQPGRSLRLSCAASGFTFSSYAMHWVRQAPGKGLEWVAVISYDGSNKYYADSVKGRFTISRDNSKNTLYLQMNSLRAEDTAVYYCARDRRGRKSAGIDYWGQGTLVTVSS, et une chaîne légère ayant la séquence d'acides aminés : 
DIQMTQSPSSLSASVGDRVTITCQASQDISNYLNWYQQKPGKAPKLLIYAASSLQ
SGVPSRFSGSGSGTDFTLTISSLQPEDFATYYCLQDYNGWTFGQGTKVDIKRAAA
EQKLISEEDLNGAA ;
b) l'anticorps comprenant une chaîne lourde ayant la séquence d'acides aminés :
MAQVQLVQSGGGLVQPGRSLRLSCAASGFSFSEYPMHWVRQAPGRGLESVAVISYDGEYQKYADSVKGRFTISRDDSKSTVYLQMNSLRPEDTAVYYCARTINNGMDVWGQGTTVT, et une chaîne légère ayant la séquence d'acides aminés : 
DIVMTQSPSSLSASVGDRVTITCRASQGIRNDLGWYQQKPGKAPELLIYGASSLQ
SGVPSRFSGSGSGTDFTLTISSLQPEDSATYYCLQDYNGWTFGQGTKLEIKRAAA
EQKLISEEDLNGAA ;
c) l'anticorps comprenant une chaîne lourde ayant la séquence d'acides aminés :
MAQVQLVESGGGLVQPGGSLRLSCAASGFTFSSYAMHWVRQAPGKGLEWVAVISYDGSNKYYADSVKGRFTISRDNSKNTLYLQMNSLRAEDTAVYYCARTVGATGAFDIWGQGTMVTVSSS, et une chaîne légère ayant la séquence d'acides aminés : 
DIVMTQSPSTVSASVGDRVIIPCRASQSIGKWLAWYQQKPGKAPKLLIYKASSLE
GWVPSRFSGSGSGTEFSLTISSLQPDDSATYVCQQSHNFPPTFGGGTKLEIKRAAA
EQKLISEEDLNGAA ;
d) l'anticorps comprenant une chaîne lourde ayant la séquence d'acides aminés :
MAQVQLVQSGGGLVQPGRSLRLSCAASGFSFSEYPMHWVRQAPGRGLESVAVISYDGEYQKYADSVKGRFTISRDDSKSTVYLQMNSLRPEDTAVYYCARTINNGMDVWGQGTTVT, et une chaîne légère ayant la séquence d'acides aminés : 
DIVMTQSPSSLSASVGDRVTITCRASQGIRNDLGWYQQKPGKAPELLIYGASSLQ
SGVPSRFSGSGSGTDFTLTISSLQPEDSATYYCLQDYNGWTFGQGTKLEIKRAAA
EQKLISEEDLNGAA ;
e) l'anticorps étant en compétition pour la liaison à EMP2 avec un anticorps selon a), b), c), ou d) ;
f) l'anticorps partageant une identité d'acides aminés de 90 % avec les régions variables des chaînes lourde et légère d'un anticorps selon a), b), c), ou d) ; ou
g) l'anticorps comprenant des séquences CDR identiques à celles d'un anticorps selon a), b), c), ou d).

2. L'anticorps pour une utilisation selon la revendication 1, ladite utilisation comprenant en outre l'administration d'au moins un agent anticancéreux supplémentaire.

3. L'anticorps pour une utilisation selon la revendication 2, où le au moins un agent anticancéreux supplémentaire
(i) est sélectionné dans le groupe constitué de médicaments de chimiothérapie à base de platine, de taxanes, d'inhibiteurs de tyrosine kinase, d'anticorps anti-EGFR, d'anticorps anti-ErbB2, et de combinaisons de ceux-ci,
(ii) comprend un inhibiteur d'EGFR, ou
(iii) comprend un inhibiteur de VEGF.

4. L'anticorps pour une utilisation selon n'importe lesquelles des revendications 1 à 3, où l'anticorps est conjugué à un fragment effecteur, par exemple un agent toxique.

5. L'anticorps pour une utilisation selon n'importe lesquelles des revendications 1 à 4, où le cancer est un cancer du sein.

6. L'anticorps pour une utilisation selon n'importe laquelle des revendications 1 à 4, où le cancer est un cancer sélectionné dans un groupe comprenant un cancer du cerveau, un cancer du côlon, un mélanome, une leucémie (par ex., LAM), un cancer du pancréas, un cancer de la prostate, un cancer de l'ovaire, un cancer du poumon, et un cancer de l'estomac.

7. L'anticorps pour une utilisation selon n'importe laquelle des revendications 1 à 6, ladite utilisation comprenant en outre l'utilisation d'un diagnostic compagnon, par exemple un anticorps anti-EMP2.

8. L'anticorps pour une utilisation selon n'importe lesquelles des revendications 1 à 3, où le cancer est un cancer de l'endomètre.

9. Une méthode in vitro de détection de cellules souches cancéreuses, la méthode comprenant : la fourniture d'un échantillon biologique dérivé d'un humain ayant ou suspecté d'avoir un cancer ; et la détection de l'expression d'EMP2 et d'un ou de plusieurs marqueurs sélectionnés dans le groupe constitué de CD44, CD133, ABCG2, et ALDH, l'expression d'EMP2 étant détectée avec un anticorps qui partage une identité d'acides aminés de 90 % avec les régions variables des chaînes lourde et légère d'un diabody comprenant :
a) une chaîne lourde ayant la séquence d'acides aminés :
MAQVQLVQSGGGVVQPGRSLRLSCAASGFTFSSYAMHWVRQAPGKGLEWVAVISYDGSNKYYADSVKGRFTISRDNSKNTLYLQMNSLRAEDTAVYYCARDRRGRKSAGIDYWGQGTLVTVSS, et une chaîne légère ayant la séquence d'acides aminés : 
DIQMTQSPSSLSASVGDRVTITCQASQDISNYLNWYQQKPGKAPKLLIYAASSLQ
SGVPSRFSGSGSGTDFTLTISSLQPEDFATYYCLQDYNGWTFGQGTKVDIKRAAA
EQKLISEEDLNGAA ;
b) une chaîne lourde ayant la séquence d'acides aminés :
MAQVQLVQSGGGLVQPGRSLRLSCAASGFSFSEYPMHWVRQAPGRGLESVAVISYDGEYQKYADSVKGRFTISRDDSKSTVYLQMNSLRPEDTAVYYCARTINNGMDVWGQGTTVT, et une chaîne légère ayant la séquence d'acides aminés : 
DIVMTQSPSSLSASVGDRVTITCRASQGIRNDLGWYQQKPGKAPELLIYGASSLQ
SGVPSRFSGSGSGTDFTLTISSLQPEDSATYYCLQDYNGWTFGQGTKLEIKRAAA
EQKLISEEDLNGAA ;
c) une chaîne lourde ayant la séquence d'acides aminés :
MAQVQLVESGGGLVQPGGSLRLSCAASGFTFSSYAMHWVRQAPGKGLEWVAVISYDGSNKYYADSVKGRFTISRDNSKNTLYLQMNSLRAEDTAVYYCARTVGATGAFDIWGQGTMVTVSSS, et une chaîne légère ayant la séquence d'acides aminés : 
DIVMTQSPSTVSASVGDRVIIPCRASQSIGKWLAWYQQKPGKAPKLLIYKASSLE
GWVPSRFSGSGSGTEFSLTISSLQPDDSATYVCQQSHNFPPTFGGGTKLEIKRAAA
EQKLISEEDLNGAA ; ou
d) une chaîne lourde ayant la séquence d'acides aminés :
MAQVQLVQSGGGLVQPGRSLRLSCAASGFSFSEYPMHWVRQAPGRGLESVAVISYDGEYQKYADSVKGRFTISRDDSKSTVYLQMNSLRPEDTAVYYCARTINNGMDVWGQGTTVT, et une chaîne légère ayant la séquence d'acides aminés :

DIVMTQSPSSLSASVGDRVTITCRASQGIRNDLGWYQQKPGKAPELLIYGASSLQ
SGVPSRFSGSGSGTDFTLTISSLQPEDSATYYCLQDYNGWTFGQGTKLEIKRAAA
EQKLISEEDLNGAA.

10. La méthode de la revendication 9, où l'expression d'EMP2 est détectée avec un anticorps comprenant :
a) une chaîne lourde ayant la séquence d'acides aminés :
MAQVQLVQSGGGVVQPGRSLRLSCAASGFTFSSYAMHWVRQAPGKGLEWVAVISYDGSNKYYADSVKGRFTISRDNSKNTLYLQMNSLRAEDTAVYYCARDRRGRKSAGIDYWGQGTLVTVSS, et une chaîne légère ayant la séquence d'acides aminés : DIQMTQSPSSLSASVGDRVTITCQASQDISNYLNWYQQKPGKAPKLLIYAASSLQ 
SGVPSRFSGSGSGTDFTLTISSLQPEDFATYYCLQDYNGWTFGQGTKVDIKRAAA
EQKLISEEDLNGAA ;
b) une chaîne lourde ayant la séquence d'acides aminés :
MAQVQLVQSGGGLVQPGRSLRLSCAASGFSFSEYPMHWVRQAPGRGLESVAVISYDGEYQKYADSVKGRFTISRDDSKSTVYLQMNSLRPEDTAVYYCARTINNGMDVWGQGTTVT, et une chaîne légère ayant la séquence d'acides aminés : 
DIVMTQSPSSLSASVGDRVTITCRASQGIRNDLGWYQQKPGKAPELLIYGASSLQ
SGVPSRFSGSGSGTDFTLTISSLQPEDSATYYCLQDYNGWTFGQGTKLEIKRAAA
EQKLISEEDLNGAA ;
c) une chaîne lourde ayant la séquence d'acides aminés :
MAQVQLVESGGGLVQPGGSLRLSCAASGFTFSSYAMHWVRQAPGKGLEWVAVISYDGSNKYYADSVKGRFTISRDNSKNTLYLQMNSLRAEDTAVYYCARTVGATGAFDIWGQGTMVTVSSS, et une chaîne légère ayant la séquence d'acides aminés : 
DIVMTQSPSTVSASVGDRVIIPCRASQSIGKWLAWYQQKPGKAPKLLIYKASSLE
GWVPSRFSGSGSGTEFSLTISSLQPDDSATYVCQQSHNFPPTFGGGTKLEIKRAAA
EQKLISEEDLNGAA ; ou
d) une chaîne lourde ayant la séquence d'acides aminés :
MAQVQLVQSGGGLVQPGRSLRLSCAASGFSFSEYPMHWVRQAPGRGLESVAVISYDGEYQKYADSVKGRFTISRDDSKSTVYLQMNSLRPEDTAVYYCARTINNGMDVWGQGTTVT, et une chaîne légère ayant la séquence d'acides aminés :

DIVMTQSPSSLSASVGDRVTITCRASQGIRNDLGWYQQKPGKAPELLIYGASSLQ
SGVPSRFSGSGSGTDFTLTISSLQPEDSATYYCLQDYNGWTFGQGTKLEIKRAAA
EQKLISEEDLNGAA.

11. La méthode de la revendication 9 ou de la revendication 10, où l'humain a ou est suspecté d'avoir un cancer du sein, par exemple un cancer du sein triple négatif, ou un cancer de l'endomètre.
